# EUROPÄISCHE PATENTSCHRIFT

(11) **EP 4 169 280 B1**
(45) Veröffentlichungstag und Bekanntmachung des Hinweises auf die Patenterteilung: **04.03.2026**
(21) Anmeldenummer: 21737350.5
(22) Anmeldetag: 21.06.2021
(51) Int. Cl.: H04W 12/06, H04W 12/08, H04W 4/80, H04W 4/02, H04W 12/50, H04B 11/00, A61M 1/14, H04L 9/40

(54) **VORRICHTUNG UND VERFAHREN ZUR AUTHENTIFIZIERUNG EINES BENUTZERS EINER MEDIZINISCHEN VORRICHTUNG**
DEVICE AND METHOD FOR THE AUTHENTICATION OF A USER OF A MEDICAL DEVICE
DISPOSITIF ET PROCÉDÉ D'AUTHENTIFICATION D'UN UTILISATEUR D'UN DISPOSITIF MÉDICAL

(30) Priorität: 22.06.2020 DE 102020207697
(43) Veröffentlichungstag der Anmeldung: 26.04.2023
(73) Patentinhaber: Fresenius Medical Care Deutschland GmbH, 61352 Bad Homburg (DE)
(72) Erfinder: MARTERSTOCK, Stefan Konrad, 61352 Bad Homburg (DE); HARTMANN, Kevin Heiko, 61352 Bad Homburg (DE)
(74) Vertreter: Ricker, Mathias
(86) Internationale Anmeldenummer: PCT/EP2021/066776
(87) Internationale Veröffentlichungsnummer: WO 2021/259837

(56) Entgegenhaltungen:
- US-A- 5 309 144
- US-A1- 2014 068 751
- US-A1- 2014 258 392
- US-A1- 2017 295 466
- US-A1- 2020 114 054

## Beschreibung

Die vorliegende Erfindung betrifft eine Vorrichtung und ein Verfahren zur Authentifizierung eines Benutzers einer medizinischen Vorrichtung. Die vorliegende Erfindung betrifft insbesondere eine medizinische Vorrichtung für ein Behandlungssystem, ein Behandlungssystem zur Behandlung eines Patienten umfassend eine medizinische Vorrichtung und eine tragbare Authentifizierungsvorrichtung, sowie ein Verfahren zur Überprüfung der Authentifikation einer tragbaren Authentifizierungsvorrichtung für ein Behandlungssystem. Hierbei dienen die medizinische Vorrichtung und das Behandlungssystem bevorzugt zur Durchführung einer Dialysebehandlung eines Patienten.

Unter medizinischen Vorrichtungen werden hier insbesondere Vorrichtungen zur Leitung, Behandlung und/oder Verteilung von Flüssigkeiten und/oder Gasen verstanden, bei denen über eine Fluidleitung Fluid zwischen einem Patienten und einer Fluidbehandlungskomponente und/oder einer Fluidquelle transportiert werden.

Unter medizinischen Vorrichtungen werden insbesondere auch Fluidbehandlungsvorrichtungen wie beispielsweise Blutbehandlungsvorrichtungen verstanden, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispiele für solche Blutbehandlungsvorrichtungen sind insbesondere Hämodialysevorrichtungen.

Die Dialyse ist ein Verfahren zur Blutreinigung von Patienten mit akuter oder chronischer Niereninsuffizienz. Grundsätzlich unterscheidet man hierbei Verfahren mit einem extrakorporalen Blutkreislauf, wie die Hämodialyse, die Hämofiltration oder die Hämodiafiltration und der Peritonealdialyse, die keinen extrakorporalen Blutkreislauf aufweist.

Die Verfahren der Hämodialyse, der Hämofiltration und der Hämodiafiltration, im Folgenden unter dem Begriff Hämodialyse zusammengefasst, werden in der Regel mit automatischen Hämodialysevorrichtungen durchgeführt, wie sie beispielsweise von der Anmelderin unter der Bezeichnung 5008 vertrieben werden.

Das Verfahren zur Peritonealdialyse wird in der Regel mit Hilfe von automatischen Peritonealdialysevorrichtungen, wie sie beispielsweise von der Anmelderin unter der Bezeichnung sleep.safe vertrieben werden, durchgeführt.

Dialysevorrichtungen, als Beispiel für komplexe medizinische Vorrichtungen, weisen einen umfangreichen Funktionsumfang zur Durchführung der Dialysebehandlung auf. Um diese Funktionen zu steuern, sind medizinische Vorrichtungen wie Dialysevorrichtungen mit zumindest einer Steuervorrichtung ausgerüstet, welche als Zentraleinheit oder Mikrokontroller ausgebildet sein kann, und welche unter Verwendung von Softwareprogrammen, die in einer Speichervorrichtung der medizinischen Vorrichtung gespeichert sind, die medizinische Vorrichtung steuert. Zur Bedienung solcher Vorrichtungen weisen diese eine Eingabe-/Ausgabevorrichtung, die beispielsweise als Touchscreen ausgebildet sein kann, oder getrennt voneinander vorgesehene Eingabe- und Ausgabevorrichtungen auf. Mittels der Eingabe-/Ausgabevorrichtung können beispielsweise durch einen Benutzer auch Patientendaten wie etwa Name, Alter, Patientenidentifikationsnummer, Patientenhistorie, und Ähnliches eingegeben und in der Speichervorrichtung gespeichert werden. In der Speichervorrichtung können auch während einer Behandlung des Patienten aufgezeichnete Betriebsparameter der medizinischen Vorrichtung automatisch und dem Patienten zugeordnet abgespeichert werden. Die in der Speichervorrichtung gespeicherten Daten, insbesondere die Patientendaten und die Betriebsparameter, können dann zu einem späteren Zeitpunkt von demselben Benutzer oder von einem anderen Benutzer mittels der Eingabe-/Ausgabevorrichtung abgerufen werden.

Die Behandlung von Patienten mittels Dialysevorrichtungen stellt einen schwerwiegenden Eingriff in den Blutkreislauf des Patienten dar. Daher ist es erforderlich, dass die entsprechenden Dialysevorrichtungen lediglich von speziell geschultem Bedienpersonal eingerichtet und eingestellt werden können. Da in einer üblichen Dialysestation Dialysevorrichtungen unterschiedlicher Bauart vorhanden sein können, kann es vorkommen, dass bestimmte Dialyseschwestern nicht auf allen Dialysevorrichtungen geschult sind und entsprechend eine Bedienung, besonders eine Einstellung kritischer Betriebsparameter, nur durch entsprechend geschultes Personal durchgeführt werden sollte.

Weiterhin sollte vermieden werden, dass die Patienten selbst, oder Besucher oder Dritte die Dialysevorrichtungen während eines Dialysevorganges absichtlich oder unbeabsichtigt manipulieren können. Darüber hinaus sollte verhindert werden, dass unbefugte Personen Zugriff auf die in der Speichervorrichtung gespeicherten Patientendaten und/oder die dem Patienten zugeordneten Betriebsparameter erhalten.

Um zu verhindern, dass eine komplexe medizinische Vorrichtung zur Behandlung eines Patienten wie etwa eine Dialysevorrichtung durch eine unbefugte Person wie etwa einen Patienten oder nicht ausreichend geschultes Personal bedient wird, werden üblicherweise Maßnahmen ergriffen, durch welche sichergestellt werden soll, dass lediglich befugte Personen Betriebsparameter der medizinischen Vorrichtung einstellen oder ändern können und Zugriff auf die in der Speichervorrichtung gespeicherten Daten erhalten. Hierzu kann es beispielsweise erforderlich sein, dass sich ein Benutzer durch Eingabe einer Benutzerkennung und einem der Benutzerkennung zugeordneten Passwort an der medizinischen Vorrichtung anmelden bzw. authentifizieren muss, bevor er Zugriff auf die in der Speichervorrichtung gespeicherten Daten erhält oder die Betriebsparameter der medizinischen Vorrichtung einstellen oder ändern kann.

Zur Erhöhung des Komforts bei der Bedienung von medizinischen Vorrichtungen werden auch tragbare Authentifizierungsvorrichtungen eingesetzt, die dazu eingerichtet sind, drahtlos mit der medizinischen Vorrichtung zu kommunizieren und ein Authentifizierungssignal, das Informationen enthält, welche den Benutzer der tragbaren Authentifizierungsvorrichtung oder die tragbare Authentifizierungsvorrichtung identifizieren, an die medizinische Vorrichtung zu senden. Hierbei besteht jedoch die Gefahr, dass sich ein unbefugter Benutzer, beispielsweise durch Durchführung eines Man-In-The-Middle-Angriffs, unberechtigt Zugang zu der medizinischen Vorrichtung verschafft und somit die Betriebsparameter der medizinischen Vorrichtung einstellen und ändern kann oder auf die in der Speichervorrichtung gespeicherten Daten zugreifen kann. Bei dem Man-In-The-Middle-Angriff schaltet sich der Angreifer mittels einer entsprechenden Vorrichtung logisch in den Kommunikationskanal zwischen der tragbaren Authentifizierungsvorrichtung und der medizinischen Vorrichtung und kann so den Datenverkehr, einschließlich des Authentifizierungssignals, zwischen der tragbaren Authentifizierungsvorrichtung und der medizinischen Vorrichtung auslesen. Auf diese Weise kann sich der Angreifer zu einem späteren Zeitpunkt unter Verwendung des ausgelesenen Authentifizierungssignals an der medizinischen Vorrichtung authentifizieren und somit die medizinische Vorrichtung steuern und/oder die Betriebsparameter der medizinischen Vorrichtung einstellen oder ändern.
Die US 2014/0258392 A1 offenbart eine Telekommunikationsvorrichtung, welche einen elektronischen Speicher, um mindestens eine Netzwerkadresse und einen Zufallszahlencode zu speichern, eine Schalleinheit, um die Netzwerkadresse und den Zufallszahlencode über Ultraschallschallwellen zu übertragen, eine Netzwerkschnittstelle zur Kommunikation über ein Datennetzwerk unter Verwendung der Netzwerkadresse und einen Prozessor zum Aufbau einer Kommunikationssitzung mit einer anderen Vorrichtung über das Datennetzwerk auf der Grundlage des Empfangs einer Antwort von der anderen Vorrichtung über das Datennetzwerk aufweist, wobei die Antwort mindestens den Zufallszahlencode enthält, und die Telekommunikationsvorrichtung die Kommunikationssitzung mit der anderen Vorrichtung nicht aufbaut, wenn der Zufallszahlencode in der Antwort fehlt oder wenn der Zufallszahlencode in der Antwort nicht mit dem in den Ultraschallwellen übertragenen Zufallszahlencode übereinstimmt.

Der vorliegenden Erfindung liegt daher die Aufgabe zugrunde, die Bediensicherheit von medizinischen Vorrichtungen und den Schutz vor unbefugtem Zugriff auf die medizinischen Vorrichtungen weiter zu verbessern und somit die Patientensicherheit weiter zu erhöhen.

Zur Lösung dieser Aufgabe dienen ein Behandlungssystem zur Behandlung eines Patienten gemäß einem der Patentansprüche 1 bis 4und ein Verfahren zur Überprüfung der Authentifikation einer tragbaren Authentifizierungsvorrichtung für ein Behandlungssystem gemäß einem der Patentansprüche 12 bis 15. Bevorzugte Ausgestaltungen der Erfindung sind Gegenstand der Unteransprüche und der vorliegenden Beschreibung der Erfindung.

Ein Behandlungssystem zur Behandlung eines Patienten gemäß einer Ausführungsform umfasst eine medizinische Vorrichtung und eine tragbare Authentifizierungsvorrichtung, wobei die medizinische Vorrichtung und die tragbare Authentifizierungsvorrichtung dazu eingerichtet sind, drahtlos miteinander zu kommunizieren, die medizinische Vorrichtung dazu eingerichtet ist, bei erfolgreich hergestellter drahtloser Kommunikationsverbindung zwischen der medizinischen Vorrichtung und der tragbaren Authentifizierungsvorrichtung ein akustisches Signal auszugeben, die tragbare Authentifizierungsvorrichtung dazu eingerichtet ist, das akustische Signal zu empfangen, basierend auf dem empfangenen akustischen Signal ein Signal zu erzeugen, das ein Signal enthält, welches dem empfangenen akustischen Signal entspricht, und das erzeugte Signal drahtlos an die medizinische Vorrichtung auszusenden, und die medizinische Vorrichtung dazu eingerichtet ist, in Abhängigkeit davon, ob sie das Signal, das das Signal enthält, welches dem von der tragbaren Authentifizierungsvorrichtung empfangenen akustischen Signal entspricht, empfängt oder nicht, zu bestimmen, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Ein Behandlungssystem zur Behandlung eines Patienten gemäß einer anderen Ausführungsform umfasst eine medizinische Vorrichtung und eine tragbare Authentifizierungsvorrichtung, wobei die medizinische Vorrichtung und die tragbare Authentifizierungsvorrichtung dazu eingerichtet sind, drahtlos miteinander zu kommunizieren, die medizinische Vorrichtung dazu eingerichtet ist, bei erfolgreich hergestellter drahtloser Kommunikationsverbindung zwischen der medizinischen Vorrichtung und der tragbaren Authentifizierungsvorrichtung drahtlos ein Signal auszusenden, das ein Signal enthält, welches einem akustischen Signal entspricht, die tragbare Authentifizierungsvorrichtung dazu eingerichtet ist, das Signal, das das Signal enthält, welches dem akustischen Signal entspricht, zu empfangen, basierend auf dem empfangenen Signal ein akustisches Signal zu erzeugen, das dem Signal, welches dem akustischen Signal entspricht, entspricht, und das erzeugte akustische Signal an die medizinische Vorrichtung auszugeben, und die medizinische Vorrichtung dazu eingerichtet ist, in Abhängigkeit davon, ob sie das akustische Signal empfängt oder nicht, zu bestimmen, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Ein Behandlungssystem zur Behandlung eines Patienten gemäß einer weiteren Ausführungsform umfasst eine medizinische Vorrichtung und eine tragbare Authentifizierungsvorrichtung, wobei die medizinische Vorrichtung und die tragbare Authentifizierungsvorrichtung dazu eingerichtet sind, drahtlos miteinander zu kommunizieren, die tragbare Authentifizierungsvorrichtung dazu eingerichtet ist, bei erfolgreich hergestellter drahtloser Kommunikationsverbindung zwischen der medizinischen Vorrichtung und der tragbaren Authentifizierungsvorrichtung drahtlos ein Signal auszusenden, das ein Signal enthält, welches einem akustischen Signal entspricht, die medizinische Vorrichtung dazu eingerichtet ist, das Signal, das das Signal enthält, welches dem akustischen Signal entspricht, zu empfangen, basierend auf dem empfangenen Signal ein akustisches Signal zu erzeugen, das dem Signal, welches dem akustischen Signal entspricht, entspricht, und das erzeugte akustische Signal an die tragbare Authentifizierungsvorrichtung auszugeben, die tragbare Authentifizierungsvorrichtung dazu eingerichtet ist, das akustische Signal zu empfangen, und bei Empfang des akustischen Signals drahtlos ein Signal an die medizinische Vorrichtung auszusenden, das angibt, dass die tragbare Authentifizierungsvorrichtung das akustische Signal empfangen hat, und die medizinische Vorrichtung dazu eingerichtet ist, in Abhängigkeit davon, ob sie das Signal, das angibt, dass die tragbare Authentifizierungsvorrichtung das akustische Signal empfangen hat, empfängt oder nicht, zu bestimmen, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Ein Behandlungssystem zur Behandlung eines Patienten gemäß einer weiteren anderen Ausführungsform umfasst eine medizinische Vorrichtung und eine tragbare Authentifizierungsvorrichtung, wobei die medizinische Vorrichtung und die tragbare Authentifizierungsvorrichtung dazu eingerichtet sind, drahtlos miteinander zu kommunizieren, die tragbare Authentifizierungsvorrichtung dazu eingerichtet ist, bei erfolgreich hergestellter drahtloser Kommunikationsverbindung zwischen der medizinischen Vorrichtung und der tragbaren Authentifizierungsvorrichtung ein vorgegebenes akustisches Signal, vorzugsweise eine vorgegebene Tonfolge, auszugeben, und die medizinische Vorrichtung dazu eingerichtet ist, in Abhängigkeit davon, ob sie das vorgegebene akustische Signal empfängt oder nicht, zu bestimmen, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Gemäß den erfindungsgemäßen Behandlungssystemen zur Behandlung eines Patienten kann bei der Authentifizierung der tragbaren Authentifizierungsvorrichtung durch die zusätzliche Verwendung eines akustischen Kommunikationskanals zu dem üblichen Kommunikationskanal mittels drahtloser Signale die funktionale Sicherheit der medizinischen Vorrichtung sichergestellt werden und ein Man-In-The-Middle-Angriff erschwert werden. Zudem wird der Benutzer durch Ausgabe des akustischen Signals durch die medizinische Vorrichtung oder die tragbare Authentifizierungsvorrichtung zusätzlich über einen stattfindenden Authentifizierungsvorgang informiert, so dass er, falls er sich nicht gerade selbst authentifiziert, gegebenenfalls Maßnahmen gegen einen Man-In-The-Middle-Angriff ergreifen kann.

Gemäß einer Ausführungsform ist das akustische Signal eine zufällige oder eine vorgegebene Tonfolge, die mehrere unterschiedliche Töne aufweisen kann, welche sequentiell ausgegeben werden, und das Signal, das das Signal enthält, welches dem akustischen Signal entspricht, kann ein Signal enthalten, das der zufälligen oder der vorgegebenen Tonfolge entspricht.

Im Falle, dass das akustische Signal die vorgegebene Tonfolge ist, kann die Tonfolge durch eine individuelle Identifikationsnummer, die die medizinische Vorrichtung eindeutig identifiziert, bestimmt sein. Auf diese Weise kann der Benutzer vorteilhaft schnell erkennen, an welcher medizinischen Vorrichtung er sich authentifiziert.

Gemäß einer Ausführungsform sind die medizinische Vorrichtung und die tragbare Authentifizierungsvorrichtung dazu eingerichtet, drahtlos über einen gemeinsamen Funkstandard miteinander zu kommunizieren und/oder sind die medizinische Vorrichtung und die tragbare Authentifizierungsvorrichtung dazu eingerichtet, drahtlos über ein Infrarotsignal miteinander zu kommunizieren.

Die medizinische Vorrichtung ist dazu eingerichtet, ein Abfragesignal zur Abfrage von Authentifizierungsinformationen drahtlos an die tragbare Authentifizierungsvorrichtung auszusenden und ein Authentifizierungssignal drahtlos zu empfangen, wobei die tragbare Authentifizierungsvorrichtung dazu eingerichtet ist zyklisch und/oder nach Empfang des Abfragesignals ein Authentifizierungssignal auszusenden, welches Informationen enthält, welche die tragbare Authentifizierungsvorrichtung identifizieren, die medizinische Vorrichtung dazu eingerichtet ist, bei Empfang des Authentifizierungssignals von der tragbaren Authentifizierungsvorrichtung eine Authentifikation durchzuführen, bei der geprüft wird, ob das Authentifizierungssignal einem in einer Berechtigungsdatenbank registrierten Benutzer oder einer in der Berechtigungsdatenbank registrierten tragbaren Authentifizierungsvorrichtung zugeordnet ist oder nicht, und ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht, und zu bestimmen, dass die Authentifizierung erfolgreich ist, wenn das Authentifizierungssignal einem in einer Berechtigungsdatenbank registrierten Benutzer oder einer in der Berechtigungsdatenbank registrierten tragbaren Authentifizierungsvorrichtung zugeordnet ist und sich die tragbare Authentifizierungsvorrichtung an einer Position, an der eine akustische Kommunikation zwischen der medizinischen Vorrichtung und der tragbaren Authentifizierungsvorrichtung möglich ist, befindet.

Die Authentifizierung erfolgt somit unter Verwendung eines zweikanaligen Authentifizierungsprozesses, der einen ersten Kommunikationskanal zur Übertragung von drahtlosen Signalen, insbesondere Datensignalen, wie etwa dem Abfragesignal zur Abfrage von Authentifizierungsinformationen und dem Authentifizierungssignal, und einen zweiten Kommunikationskanal zur Übertragung des akustischen Signal nutzt. Auf diese Weise kann ein Man-In-The-Middle-Angriff, der nur auf einem der beiden Kommunikationskanäle durchgeführt wird, verhindert werden und somit die Patientensicherheit erhöht werden.

Die Berechtigungsdatenbank kann in einer Speichervorrichtung der medizinischen Vorrichtung oder in einer mit einer Steuervorrichtung der medizinischen Vorrichtung in Kommunikationsverbindung stehenden Speichervorrichtung gespeichert sein, wobei in der Berechtigungsdatenbank für jede einer Vielzahl von unterschiedlichen tragbaren Authentifizierungsvorrichtungen ein individuelles Codewort zusammen mit einer oder mehreren Berechtigungen gespeichert sind, die dem jeweiligen individuellen Codewort zugeordnet sind. Bei einer bevorzugten Ausführungsform kann in der Berechtigungsdatenbank auch für jeden individuellen Benutzer ein individuelles Codewort zusammen mit einer oder mehreren Berechtigungen gespeichert sein, die dem jeweiligen individuellen Codewort zugeordnet sind. Hierbei enthält das Authentifizierungssignal, das von der tragbaren Authentifizierungsvorrichtung gesendet wird, das jeweilige individuelle Codewort. Auf diese Weise kann über eine vorgegebene Zuordnung der tragbaren Authentifizierungsvorrichtung zu einem bestimmten Benutzer zu festgelegten Zeiten oder durch die Zuordnung des Codeworts zu dem Benutzer der die medizinische Vorrichtung bedienende Benutzer identifiziert werden. Die Berechtigungen können eine Berechtigung, die den jeweiligen Benutzer, beispielsweise einen Arzt oder eine voll ausgebildete und entsprechend zertifizierte Dialyseschwester, dazu berechtigt, auf sämtliche Funktionen der medizinischen Vorrichtung zuzugreifen, und jeweilige Berechtigungen umfassen, die den jeweiligen Benutzer lediglich dazu berechtigen, auf bestimmte, auf den Ausbildungsstand des Benutzer abgestimmte Funktionen der medizinischen Vorrichtung zuzugreifen.

Insbesondere bestimmt die medizinische Vorrichtung, wenn die medizinische Vorrichtung bestimmt, dass die Authentifizierung erfolgreich ist, dass der Benutzer, dem das entsprechende Authentifizierungssignal zugeordnet ist, für die Bedienung der spezifischen medizinischen Vorrichtung zugelassen ist, und gibt einen Zugriff auf die medizinische Vorrichtung im Rahmen der dem Authentifizierungssignal in der Berechtigungsdatenbank zugeordneten Berechtigungen frei.

Gemäß einer bevorzugten Ausführungsform weist die medizinische Vorrichtung ferner eine Speichervorrichtung, in der Patientendaten und/oder den Patientendaten zugeordnete Betriebsparameter der medizinischen Vorrichtung, die während einer Behandlung des Patienten aufgezeichnet wurden, speicherbar oder gespeichert sind, und eine Eingabe-/Ausgabevorrichtung mit einem Display zur Anzeige der Patientendaten und/oder der den Patientendaten zugeordneten Betriebsparameter der medizinischen Vorrichtung auf, wobei ein Benutzer mittels einer Eingabe unter Verwendung der Eingabe-/Ausgabevorrichtung nur bewirken kann, dass die in der Speichervorrichtung gespeicherten Patientendaten und/oder die den Patientendaten zugeordneten Betriebsparameter der medizinischen Vorrichtung, die in der Speichervorrichtung gespeichert sind, auf dem Display der Eingabe-/Ausgabevorrichtung angezeigt werden, wenn die medizinische Vorrichtung bestimmt, dass die Authentifizierung erfolgreich ist.

Somit kann gemäß dieser bevorzugten Ausführungsform ein unbefugter Zugriff auf die in der Speichervorrichtung der medizinischen Vorrichtung gespeicherten Patientendaten wie etwa Name, Alter, Patientenidentifikationsnummer, Patientenhistorie, und Ähnliches, sowie ein unbefugter Zugriff auf während einer Behandlung des Patienten aufgezeichnete und in der Speichervorrichtung der medizinischen Vorrichtung abgespeicherte, dem Patienten zugeordnete Betriebsparameter der medizinischen Vorrichtung verhindert oder zumindest erschwert werden.

Bei einer besonders bevorzugten Ausführungsform können die Patientendaten und/oder die den Patientendaten zugeordneten Betriebsparameter, die in der Speichervorrichtung der medizinischen Vorrichtung gespeichert sind, von einem Benutzer mittels der drahtlosen Kommunikationsverbindung zwischen der medizinischen Vorrichtung und der tragbaren Authentifizierungsvorrichtung nur in eine Speichereinrichtung der tragbaren Authentifizierungsvorrichtung übertragen werden, wenn die medizinische Vorrichtung bestimmt, dass die Authentifizierung erfolgreich ist.

Gemäß einer anderen bevorzugten Ausführungsform weist die medizinische Vorrichtung eine Speichervorrichtung auf, in der eine individuelle Identifikationsnummer gespeichert ist, die die medizinische Vorrichtung eindeutig identifiziert, wobei die medizinische Vorrichtung dazu eingerichtet ist, in dem Signal, das ein Signal enthält, welches dem akustischen Signal entspricht, ferner ein Signal auszusenden, welches der in der Speichervorrichtung gespeicherten individuellen Identifikationsnummer entspricht, und die tragbare Authentifizierungsvorrichtung eine Eingabe-/Ausgabevorrichtung mit einem Display aufweist und dazu eingerichtet ist, nach Empfang des von der medizinischen Vorrichtung ausgesendeten Signals die individuelle Identifikationsnummer auf dem Display auszugeben. Durch das Ausgeben der individuellen Identifikationsnummer auf dem Display der tragbaren Authentifizierungsvorrichtung kann der Benutzer die medizinische Vorrichtung identifizieren und gegebenenfalls einen Zugriff auf die medizinische Vorrichtung durch einen unbefugten Benutzer verhindern.

Bei dieser anderen bevorzugten Ausführungsform ist die medizinische Vorrichtung dazu eingerichtet, ein Abfragesignal zur Abfrage von Authentifizierungsinformationen drahtlos an die tragbare Authentifizierungsvorrichtung auszusenden und ein Authentifizierungssignal drahtlos zu empfangen, ist die tragbare Authentifizierungsvorrichtung dazu eingerichtet zyklisch und/oder nach Empfang des Abfragesignals ein Authentifizierungssignal auszusenden, welches Informationen enthält, welche die tragbare Authentifizierungsvorrichtung identifizieren. Dabei ist die medizinische Vorrichtung dazu eingerichtet, bei Empfang des Authentifizierungssignals von der tragbaren Authentifizierungsvorrichtung eine Authentifikation durchzuführen, bei der geprüft wird, ob das Authentifizierungssignal einem in einer Berechtigungsdatenbank registrierten Benutzer oder einer in der Berechtigungsdatenbank registrierten tragbaren Authentifizierungsvorrichtung zugeordnet ist oder nicht, und ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht, und zu bestimmen, dass die Authentifizierung erfolgreich ist, wenn das Authentifizierungssignal einem in einer Berechtigungsdatenbank registrierten Benutzer oder einer in der Berechtigungsdatenbank registrierten tragbaren Authentifizierungsvorrichtung zugeordnet ist und sich die tragbare Authentifizierungsvorrichtung an einer Position, an der eine akustische Kommunikation zwischen der medizinischen Vorrichtung und der tragbaren Authentifizierungsvorrichtung möglich ist, befindet, wobei in der Speichervorrichtung Patientendaten und/oder den Patientendaten zugeordnete Betriebsparameter der medizinischen Vorrichtung, die während einer Behandlung des Patienten aufgezeichnet wurden, speicherbar oder gespeichert sind, und die medizinische Vorrichtung ferner eine Eingabe-/Ausgabevorrichtung mit einem Display zur Anzeige der Patientendaten und/oder der den Patientendaten zugeordneten Betriebsparameter der medizinischen Vorrichtung aufweist, wobei ein Benutzer mittels einer Eingabe unter Verwendung der Eingabe-/Ausgabevorrichtung nur bewirken kann, dass die in der Speichervorrichtung gespeicherten Patientendaten und/oder die den Patientendaten zugeordneten Betriebsparameter der medizinischen Vorrichtung, die in der Speichervorrichtung gespeichert sind, auf dem Display der Eingabe-/Ausgabevorrichtung angezeigt werden, wenn die medizinische Vorrichtung bestimmt, dass die Authentifizierung erfolgreich ist.

Bevorzugt können die Patientendaten und/oder die den Patientendaten zugeordneten Betriebsparameter, die in der Speichervorrichtung der medizinischen Vorrichtung gespeichert sind, von einem Benutzer mittels der drahtlosen Kommunikationsverbindung zwischen der medizinischen Vorrichtung und der tragbaren Authentifizierungsvorrichtung in die Speichereinrichtung der tragbaren Authentifizierungsvorrichtung lediglich übertragen werden, wenn die medizinische Vorrichtung bestimmt, dass die Authentifizierung erfolgreich ist.

Bei einer besonders bevorzugten Ausführungsform ist die tragbare Authentifizierungsvorrichtung als ein Mobiltelefon, insbesondere als ein Smartphone, oder als ein tragbarer Computer ausgebildet ist. Die Ausbildung der tragbaren Authentifizierungsvorrichtung als ein Smartphone ist in zweierlei Hinsicht vorteilhaft. Zum einen ist in einem Smartphone bereits ein Mikrophon und ein Lautsprecher verbaut, und zum anderen werden Smartphones von ihren Benutzern üblicherweise stets mit sich geführt, so dass das Mitführen einer weiteren Vorrichtung zur Authentifizierung an der medizinischen Vorrichtung entfallen kann.

Gemäß einer Ausführungsform ist die medizinische Vorrichtung eine Dialysevorrichtung, welche zur Durchführung einer Dialysebehandlung eingerichtet ist.

Eine medizinische Vorrichtung gemäß einer nicht beanspruchten Ausführungsform für ein Behandlungssystem ist dazu eingerichtet, drahtlos mit einer tragbaren Authentifizierungsvorrichtung zu kommunizieren und bei erfolgreich hergestellter drahtloser Kommunikationsverbindung zwischen der medizinischen Vorrichtung und der tragbaren Authentifizierungsvorrichtung ein akustisches Signal auszugeben, und in Abhängigkeit davon, ob die medizinische Vorrichtung ein von der tragbaren Authentifizierungsvorrichtung drahtlos ausgesendetes Signal, das ein Signal enthält, welches dem ausgegebenen akustischen Signal entspricht, empfängt oder nicht, zu bestimmen, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Eine medizinische Vorrichtung gemäß einer anderen nicht beanspruchten Ausführungsform für ein Behandlungssystem ist dazu eingerichtet, drahtlos mit einer tragbaren Authentifizierungsvorrichtung zu kommunizieren und bei erfolgreich hergestellter drahtloser Kommunikationsverbindung zwischen der medizinischen Vorrichtung und der tragbaren Authentifizierungsvorrichtung drahtlos ein Signal auszusenden, das ein Signal enthält, welches einem akustischen Signal entspricht, und in Abhängigkeit davon, ob die medizinische Vorrichtung ein von der tragbaren Authentifizierungsvorrichtung ausgegebenes akustisches Signal, das dem Signal, welches dem akustischen Signal entspricht, entspricht, empfängt oder nicht, zu bestimmen, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Eine medizinische Vorrichtung gemäß einer weiteren nicht beanspruchten Ausführungsform für ein Behandlungssystem ist dazu eingerichtet, drahtlos mit einer tragbaren Authentifizierungsvorrichtung zu kommunizieren und bei erfolgreich hergestellter drahtloser Kommunikationsverbindung zwischen der medizinischen Vorrichtung und der tragbaren Authentifizierungsvorrichtung ein von der tragbaren Authentifizierungsvorrichtung drahtlos ausgesendetes Signal, das ein Signal enthält, welches einem akustischen Signal entspricht, zu empfangen, basierend auf dem empfangenen Signal ein akustisches Signal zu erzeugen, das dem Signal, welches dem akustischen Signal entspricht, entspricht, und das erzeugte akustische Signal an die tragbare Authentifizierungsvorrichtung auszugeben, und in Abhängigkeit davon, ob die medizinische Vorrichtung ein von der tragbaren Authentifizierungsvorrichtung drahtlos ausgesendetes Signal, das angibt, dass die tragbare Authentifizierungsvorrichtung das akustische Signal empfangen hat, empfängt oder nicht, zu bestimmen, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Eine medizinische Vorrichtung gemäß einer weiteren anderen nicht beanspruchten Ausführungsform für ein Behandlungssystem ist dazu eingerichtet, drahtlos mit einer tragbaren Authentifizierungsvorrichtung zu kommunizieren und bei erfolgreich hergestellter drahtloser Kommunikationsverbindung zwischen der medizinischen Vorrichtung und der tragbaren Authentifizierungsvorrichtung ein von der tragbaren Authentifizierungsvorrichtung ausgegebenes vorgegebenes akustisches Signal, vorzugsweise eine vorgegebene Tonfolge, zu empfangen, und in Abhängigkeit davon, ob die medizinische Vorrichtung das vorgegebene akustische Signal empfängt oder nicht, zu bestimmen, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Ein Verfahren gemäß einer Ausführungsform zur Überprüfung der Authentifikation einer tragbaren Authentifizierungsvorrichtung für ein vorangehend beschriebenes Behandlungssystem umfasst ein Ausgeben eines akustischen Signals durch die medizinische Vorrichtung, ein Empfangen des akustischen Signals durch die tragbare Authentifizierungsvorrichtung, basierend auf dem empfangenen akustischen Signal ein Erzeugen, durch die tragbare Authentifizierungsvorrichtung, eines Signals, das ein Signal enthält, welches dem empfangenen akustischen Signal entspricht, und ein drahtloses Aussenden des erzeugten Signals an die medizinische Vorrichtung durch die tragbare Authentifizierungsvorrichtung, und ein Bestimmen, durch die medizinische Vorrichtung, in Abhängigkeit davon, ob sie das Signal, das das Signal enthält, welches dem von der tragbaren Authentifizierungsvorrichtung empfangenen akustischen Signal entspricht, empfängt oder nicht, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Ein Verfahren gemäß einer anderen Ausführungsform zur Überprüfung der Authentifikation einer tragbaren Authentifizierungsvorrichtung für ein vorangehend beschriebenes Behandlungssystem umfasst ein drahtloses Aussenden eines Signals, das ein Signal enthält, welches einem akustischen Signal entspricht, durch die medizinische Vorrichtung, ein Empfangen, durch die tragbare Authentifizierungsvorrichtung, des Signals, welches dem akustischen Signal entspricht, basierend auf dem empfangenen Signal ein Erzeugen, durch die tragbare Authentifizierungsvorrichtung, eines akustischen Signals, das dem Signal, welches dem akustischen Signal entspricht, entspricht, und ein Ausgeben des erzeugten akustischen Signals an die medizinische Vorrichtung, und ein Bestimmen, durch die medizinische Vorrichtung, in Abhängigkeit davon, ob sie das akustische Signal empfängt oder nicht, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Ein Verfahren gemäß einer weiteren Ausführungsform zur Überprüfung der Authentifikation einer tragbaren Authentifizierungsvorrichtung für ein Behandlungssystem umfasst ein drahtloses Aussenden eines Signals, das ein Signal enthält, welches einem akustischen Signal entspricht, durch die tragbare Authentifizierungsvorrichtung, ein Empfangen, durch die medizinische Vorrichtung, des Signals, das das Signal enthält, welches dem akustischen Signal entspricht, basierend auf dem empfangenen Signal Erzeugen, durch die medizinische Vorrichtung, eines akustischen Signals, das dem Signal, welches dem akustischen Signal entspricht, entspricht, und Ausgeben des erzeugten akustischen Signals an die tragbare Authentifizierungsvorrichtung durch die medizinische Vorrichtung, ein drahtloses Aussenden eines Signals, das angibt, dass die tragbare Authentifizierungsvorrichtung das akustische Signal empfangen hat, durch die tragbare Authentifizierungsvorrichtung, an die medizinische Vorrichtung, wenn die tragbare Authentifizierungsvorrichtung das akustische Signal von der medizinischen Vorrichtung empfangen hat, und ein Bestimmen, durch die medizinische Vorrichtung, in Abhängigkeit davon, ob sie das Signal, das angibt, dass die tragbare Authentifizierungsvorrichtung das akustische Signal empfangen hat, empfängt oder nicht, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Ein Verfahren gemäß einer weiteren anderen Ausführungsform zur Überprüfung der Authentifikation einer tragbaren Authentifizierungsvorrichtung für ein Behandlungssystem umfasst ein Ausgeben eines vorgegebenen akustischen Signals, vorzugsweise einer vorgegebenen Tonfolge, durch die tragbare Authentifizierungsvorrichtung, und ein Bestimmen, durch die medizinische Vorrichtung, in Abhängigkeit davon, ob sie das vorgegebene akustische Signal empfängt oder nicht, ob sich die tragbare Authentifizierungsvorrichtung an einer Position, an der eine akustische Kommunikation zwischen der medizinischen Vorrichtung und der tragbaren Authentifizierungsvorrichtung möglich ist, befindet oder nicht.

Weitere mögliche bevorzugte Ausgestaltungen des erfindungsgemäßen Verfahrens lassen sich aus der Beschreibung des erfindungsgemäßen Systems und von dessen bevorzugten Ausgestaltungen ableiten.

Weitere bevorzugte Ausgestaltungen des erfindungsgemäßen Systems und des erfindungsgemäßen Verfahrens ergeben sich aus der nachfolgenden Beschreibung der Ausführungsbeispiele in Zusammenhang mit der Figur und deren Beschreibung. Gleiche Bauteile der Ausführungsbeispiele werden im Wesentlichen durch gleiche Bezugszeichen gekennzeichnet, falls dies nicht anders beschrieben wird oder sich nicht anders aus dem Kontext ergibt. Es zeigt:

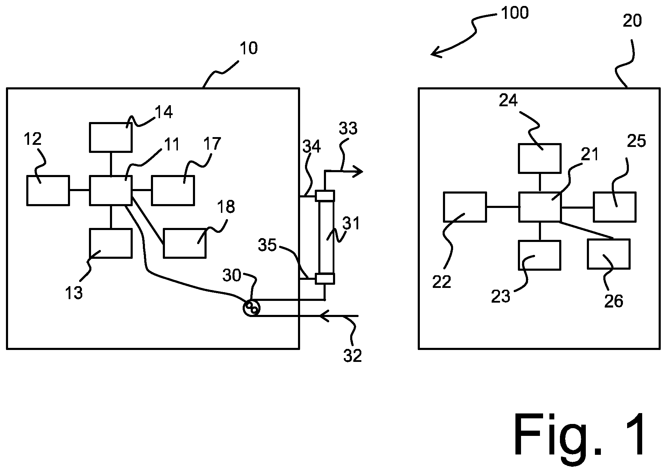

Fig. 1 schematisch ein Behandlungssystem gemäß einer Ausführungsform mit einer medizinischen Vorrichtung und einer Authentifizierungsvorrichtung.

Fig. 1 veranschaulicht ein Behandlungssystem für einen Patienten gemäß einer Ausführungsform, das üblicherweise in einer klinischen Einrichtung, beispielsweise in einer Dialysestation, in der eine Vielzahl medizinischer Vorrichtungen zur Behandlung von Patienten vorgesehen sind, verwendet wird. Das Behandlungssystem 100 weist eine medizinische Vorrichtung 10 zur Behandlung eines Patienten aus der Vielzahl medizinischer Vorrichtungen zur Behandlung eines Patienten und eine tragbare Authentifizierungsvorrichtung 20 auf, die von einem Bediener bzw. Benutzer der medizinischen Vorrichtung 10, beispielsweise einem Arzt oder einem Mitglied des klinischen Personals, das zur Bedienung der medizinischen Vorrichtung 10 berechtigt ist, mit sich geführt wird.

Die medizinische Vorrichtung 10 ist als eine Hämodialysevorrichtung bzw. als eine Dialysemaschine mit einer Steuervorrichtung 11, sowie einer Kommunikationsvorrichtung 12, einer Speichervorrichtung 13 und einer Eingabe-/Ausgabevorrichtung 14 mit einem Display, beispielsweise in Form eines Touchscreens, die mit der Steuervorrichtung 11 verbunden sind, ausgebildet. Bei anderen, nicht gezeigten Ausführungsformen kann die medizinische Vorrichtung auch als eine Peritonealdialysevorrichtung oder als eine Vorrichtung zur Behandlung von Patienten mit akuter Nierenschädigung durch eine kontinuierliche Nierenersatztherapie ausgebildet sein, welche ebenfalls eine Steuervorrichtung, sowie eine Kommunikationsvorrichtung, eine Speichervorrichtung und eine Eingabe/Ausgabevorrichtung, die mit der Steuervorrichtung verbunden sind, aufweisen.

Die Steuervorrichtung 11, die beispielsweise als Zentraleinheit oder als Mikrokontroller ausgebildet sein kann, ist dazu eingerichtet, beispielsweise mittels auf der Steuervorrichtung 11 ablaufender Softwareprogramme, welche in der Speichervorrichtung 13 gespeichert sind, die Komponenten der medizinischen Vorrichtung 10 zu steuern und in Abhängigkeit von einer Eingabe durch einen authentifizierten Benutzer mittels der Eingabe-/Ausgabevorrichtung 14 verschiedene Funktionen der medizinischen Vorrichtung 10 aufzurufen bzw. zu steuern und Betriebsparameter der medizinischen Vorrichtung 10 einzustellen.

Zur Dialysebehandlung weist die als Hämodialysevorrichtung ausgebildete medizinische Vorrichtung 20 insbesondere eine mit der Steuervorrichtung 11 verbundene Blutpumpe 30 und einen Dialysefilter 31 auf, die im Falle der Behandlung eines nicht dargestellten Patienten Bestandteile eines extrakorporalen Blutkreislaufs sind, welcher zudem eine arterielle Blutleitung 32 zur Ableitung von Blut des Patienten, und eine venöse Leitung 33 zur Rückführung des Bluts in den Patienten aufweist. Im Betrieb wird das Blut von dem Patienten über die arterielle Blutleitung 32 der Blutpumpe 30 zugeführt, und von dieser durch den Dialysefilter 31 gepumpt, welcher eine semipermeable Membran aufweist, die den extrakorporalen Blutkreislauf von einem Dialysatkreislauf semipermeabel trennt. Über mit dem Dialysefilter 31 verbundenen Dialysatleitungen 34, 35 wird Dialysat durch den Dialysefilter 31 gepumpt, in dem über die semipermeable Membran des Dialysefilters 31 ein diffusiver Stoffaustausch mit dem Blut des Patienten stattfindet. Durch den Aufbau eines Druckgradienten von der Blutseite des Dialysefilters zur Dialysatseite des Dialysefilters 31 derart, dass ein Unterdruck auf der Dialysatseite vorherrscht, wird Plasmawasser aus dem Blut in das Dialysat abgepresst, wodurch das Blut des Patienten entwässert wird.

Um die Durchführung einer Authentifizierung eines Benutzers zu ermöglichen, ist in der Speichervorrichtung 13 der medizinischen Vorrichtung 10 oder in einer nicht dargestellten externen, mit der Steuervorrichtung 11 in Kommunikationsverbindung stehenden Speichervorrichtung, eine Berechtigungsdatenbank gespeichert, in der für jede der in der klinischen Einrichtung verwendeten tragbaren Authentifizierungsvorrichtungen 20 ein individuelles Codewort zusammen mit einer oder mehreren Berechtigungen gespeichert sind, die dem jeweiligen individuellen Codewort zugeordnet sind. Bei einer bevorzugten Ausführungsform kann in der Berechtigungsdatenbank auch für jeden individuellen Benutzer ein individuelles Codewort zusammen mit einer oder mehreren Berechtigungen gespeichert sein, die dem jeweiligen individuellen Codewort zugeordnet sind. Auf diese Weise kann über eine vorgegebene Zuordnung der tragbaren Authentifizierungsvorrichtung 20 zu einem bestimmten Benutzer zu festgelegten Zeiten oder durch die Zuordnung des Codeworts zu dem Benutzer der die medizinische Vorrichtung 10 bedienende Benutzer identifiziert werden. Die Berechtigungen können eine Berechtigung, die den jeweiligen Benutzer, beispielsweise einen Arzt oder eine voll ausgebildete und entsprechend zertifizierte Dialyseschwester, dazu berechtigt, auf sämtliche Funktionen der medizinischen Vorrichtung zuzugreifen, und jeweilige Berechtigungen umfassen, die den jeweiligen Benutzer lediglich dazu berechtigen, auf bestimmte, auf den Ausbildungsstand des Benutzer abgestimmte Funktionen der medizinischen Vorrichtung 10 zuzugreifen.

Die tragbare Authentifizierungsvorrichtung 20 weist eine Steuereinrichtung 21, eine Kommunikationseinrichtung 22, eine Speichereinrichtung 23, eine Eingabe-/Ausgabeeinrichtung 24 wie etwa einen Touchscreen, einen Lautsprecher 25 und ein Mikrofon 26 auf. Dabei kann die tragbare Authentifizierungsvorrichtung 20 beispielsweise als ein tragbarer Computer, beispielsweise als ein Tablet, oder bei einer bevorzugten Ausführungsform als ein Mobilfunkgerät, insbesondere als ein Smartphone ausgebildet sein. Die Ausbildung der tragbaren Authentifizierungsvorrichtung 20 als ein Smartphone ist in zweierlei Hinsicht vorteilhaft. Zum einen ist in einem Smartphone bereits ein Mikrophon und ein Lautsprecher verbaut, und zum anderen werden Smartphones von ihren Benutzern üblicherweise stets mit sich geführt, so dass das Mitführen einer weiteren Vorrichtung zur Authentifizierung an der medizinischen Vorrichtung 10 entfallen kann.

Die medizinische Vorrichtung 10 ist dazu eingerichtet, mittels der Kommunikationsvorrichtung 12 unter Steuerung durch die Steuervorrichtung 11 mit der tragbaren Authentifizierungsvorrichtung 20 durch Aussenden von Signalen, insbesondere Datensignalen, und Empfangen von Signalen, insbesondere Datensignalen, drahtlos zu kommunizieren. Die tragbare Authentifizierungsvorrichtung 20 ist dazu eingerichtet, mittels der Kommunikationseinrichtung 22 durch Aussenden von Signalen, insbesondere Datensignalen, und Empfangen von Signalen, insbesondere Datensignalen, drahtlos mit der medizinischen Vorrichtung 10 zu kommunizieren. Dementsprechend sind die Kommunikationseinrichtung 22 und die tragbare Authentifizierungsvorrichtung 20 dazu eingerichtet, mit den gleichen Signaltypen wie die Kommunikationsvorrichtung 12 und die medizinische Vorrichtung 10 zu kommunizieren, also beispielsweise im gleichen Frequenzband unter Verwendung des gleichen Kommunikationsprotokolls. Die drahtlose Kommunikation zwischen der Kommunikationsvorrichtung 12 der medizinischen Vorrichtung 10 und der Kommunikationseinrichtung 22 der tragbaren Authentifizierungsvorrichtung 20 kann unter Verwendung bekannter Kommunikationsstandards durchgeführt werden. Beispielsweise kann die drahtlose Kommunikation über Funkprotokolle wie etwa dem zigbee Standard, WLAN, Bluetooth und anderen bekannten Funkprotokollen erfolgen.

Die medizinische Vorrichtung 10 weist weiterhin einen Lautsprecher 17 sowie ein Mikrofon 18 auf, welche ebenfalls mit der Steuervorrichtung 11 verbunden sind. Im Falle, dass von der Steuervorrichtung 11 der medizinischen Vorrichtung 10 während des Betriebs eine Fehlfunktion einer Komponente der medizinischen Vorrichtung 10 festgestellt wird, kann der Lautsprecher 17 beispielsweise zur Ausgabe eines Alarmtons verwendet werden, um den Benutzer über die Fehlfunktion zu informieren. Das Mikrofon 18 kann beispielsweise als zusätzliche Eingabevorrichtung dienen, mit der Befehle an die Steuervorrichtung 11 bzw. die medizinische Vorrichtung 10 in Form von Sprache eingegeben werden können.

Bevorzugt erfolgt die Authentifizierung eines Benutzers unter Verwendung eines zweikanaligen Authentifizierungsprozesses, der einen ersten Kommunikationskanal zur Übertragung von drahtlosen Signalen, insbesondere Datensignalen, und einen zweiten Kommunikationskanal zur Übertragung eines akustischen Signals umfasst.

Zur Authentifizierung unter Verwendung des ersten Kommunikationskanals sendet die medizinische Vorrichtung 10 gemäß einer Ausführungsform, nachdem eine drahtlose Kommunikationsverbindung zwischen der medizinischen Vorrichtung 10 und der tragbaren Authentifizierungsvorrichtung 20 erfolgreich hergestellt wurde, mittels der Kommunikationsvorrichtung 12 unter Steuerung durch die Steuervorrichtung 11 ein Abfragesignal, insbesondere ein Datensignal, zur Abfrage von Authentifizierungsinformationen drahtlos an die tragbare Authentifizierungsvorrichtung 20 aus. Bei Empfang des Abfragesignals sendet die tragbare Authentifizierungsvorrichtung 20 mittels der Kommunikationseinrichtung 22, unter Steuerung durch die Steuereinrichtung 21, ein Authentifizierungssignal, das das Codewort bzw. Informationen enthält, welche die tragbare Authentifizierungsvorrichtung 20 identifizieren, und die in der Speichereinrichtung 23 gespeichert sind, an die medizinische Vorrichtung 10 aus. Bei Empfang des Authentifizierungssignals prüft die medizinische Vorrichtung 10 anhand der Berechtigungsdatenbank, ob es sich bei der tragbaren Authentifizierungsvorrichtung 20 um eine in der Berechtigungsdatenbank registrierte tragbare Authentifizierungsvorrichtung 20 handelt oder nicht, und kann somit feststellen, ob der Benutzer, dem das entsprechende Authentifizierungssignal zugeordnet ist, prinzipiell für die Bedienung der spezifischen medizinischen Vorrichtung 10 zugelassen ist oder nicht.

Gemäß einer anderen Ausführungsform sendet die tragbare Authentifizierungsvorrichtung 20 bei der Authentifizierung unter Verwendung des ersten Kommunikationskanals, nachdem eine drahtlose Kommunikationsverbindung zwischen der medizinischen Vorrichtung 10 und der tragbaren Authentifizierungsvorrichtung 20 erfolgreich hergestellt wurde, das Authentifizierungssignal, das das Codewort bzw. Informationen enthält, welche die tragbare Authentifizierungsvorrichtung 20 identifizieren, und die in der Speichereinrichtung 23 gespeichert sind, periodisch an die medizinische Vorrichtung 10 aus. Bei Empfang des Authentifizierungssignals prüft die medizinische Vorrichtung 10 anhand der Berechtigungsdatenbank, ob es sich bei der tragbaren Authentifizierungsvorrichtung 20 um eine in der Berechtigungsdatenbank registrierte tragbare Authentifizierungsvorrichtung 20 handelt oder nicht, und kann somit feststellen, ob der Benutzer, dem das entsprechende Authentifizierungssignal zugeordnet ist, prinzipiell für die Bedienung der spezifischen medizinischen Vorrichtung 10 zugelassen ist oder nicht.

Zur Authentifizierung unter Verwendung des zweiten Kommunikationskanals ist die medizinische Vorrichtung 10 gemäß einer Ausführungsform dazu eingerichtet, ein akustisches Signal, beispielsweise eine vorgegebene oder zufällige Tonfolge, die mehrere unterschiedliche Töne umfassen kann, mittels des Lautsprechers 17 auszugeben, und die tragbare Authentifizierungsvorrichtung 20, insbesondere deren Steuereinrichtung 21, ist dazu eingerichtet, bei Empfang des akustischen Signals mittels des Mikrofons 26 basierend auf dem empfangenen akustischen Signal ein Signal, insbesondere ein Datensignal, zu erzeugen, das ein Signal enthält, welches dem empfangenen akustischen Signal entspricht, und das erzeugte Signal mittels der Kommunikationseinrichtung 22 drahtlos an die medizinische Vorrichtung 10 auszusenden.

Da das von der medizinischen Vorrichtung 10 ausgesendete akustische Signal nur von der tragbaren Authentifizierungsvorrichtung 20 empfangbar ist, wenn diese sich an einer Position, an der eine akustische Kommunikation zwischen der medizinischen Vorrichtung 10 und der tragbaren Authentifizierungsvorrichtung 20 möglich ist, befindet, bestimmt die medizinische Vorrichtung 10 anhand des Empfangs des Signals, das das Signal enthält, welches dem von der tragbaren Authentifizierungsvorrichtung 20 empfangenen akustischen Signal entspricht, dass sich die tragbare Authentifizierungsvorrichtung 20 an einer Position, an der eine akustische Kommunikation zwischen der medizinischen Vorrichtung 10 und der tragbaren Authentifizierungsvorrichtung 20 möglich ist, befindet.

Falls die medizinische Vorrichtung 10 nach Ablauf einer vorgegebenen Zeitdauer nach Aussendung des akustischen Signals von der tragbaren Authentifizierungsvorrichtung 20 hingegen kein Signal empfängt, das das Signal enthält, welches dem empfangenen akustischen Signal entspricht, bestimmt die medizinische Vorrichtung 10, dass sich die tragbare Authentifizierungsvorrichtung 20 nicht an einer Position, an der eine akustische Kommunikation zwischen der medizinischen Vorrichtung 10 und der tragbaren Authentifizierungsvorrichtung 20 möglich ist, befindet.

Gemäß einer anderen Ausführungsform ist die medizinische Vorrichtung 10 dazu eingerichtet, ein Signal, insbesondere Datensignal, mittels der Kommunikationsvorrichtung 12 drahtlos auszusenden, das ein Signal enthält, welches einem akustischen Signal, beispielsweise einer vorgegebenen oder zufälligen Tonfolge, entspricht, und die tragbare Authentifizierungsvorrichtung 20, insbesondere deren Steuereinrichtung 21, ist dazu eingerichtet, bei Empfang des Signals mittels der Kommunikationseinrichtung 22, das das Signal enthält, welches dem akustischen Signal entspricht, basierend auf dem empfangenen Signal ein akustisches Signal zu erzeugen, das dem Signal, welches dem akustischen Signal entspricht, entspricht, und das erzeugte akustische Signal mittels des Lautsprechers 25 auszugeben.

Da das von der tragbaren Authentifizierungsvorrichtung 20 ausgesendete akustische Signal nur von dem Mikrophon 18 der medizinischen Vorrichtung 10 empfangbar ist, wenn sich die tragbare Authentifizierungsvorrichtung 20 an einer Position, an der eine akustische Kommunikation zwischen der medizinischen Vorrichtung 10 und der tragbaren Authentifizierungsvorrichtung 20 möglich ist, befindet, bestimmt die medizinische Vorrichtung 10 anhand des Empfangs des akustischen Signals, dass sich die tragbare Authentifizierungsvorrichtung 20 an einer Position, an der eine akustische Kommunikation zwischen der medizinischen Vorrichtung 10 und der tragbaren Authentifizierungsvorrichtung 20 möglich ist, befindet.

Falls die medizinische Vorrichtung 10 hingegen nach Ablauf einer vorgegebenen Zeitdauer nach Aussendung des Signals, das das Signal enthält, welches dem akustischen Signal entspricht, kein akustisches Signal empfängt, das dem Signal, welches dem akustischen Signal entspricht, entspricht, bestimmt die medizinische Vorrichtung 10, dass sich die tragbare Authentifizierungsvorrichtung 20 nicht an einer Position, an der eine akustische Kommunikation zwischen der medizinischen Vorrichtung 10 und der tragbaren Authentifizierungsvorrichtung 20 möglich ist, befindet.

Ein Vorteil dieser Ausführungsform besteht darin, dass im Falle, dass sich der Benutzer der tragbaren Authentifizierungsvorrichtung 20 nicht in unmittelbarer Nähe der medizinischen Vorrichtung 10 befindet, beispielsweise im Falle eines Man-In-The-Middle-Angriffs, der Benutzer durch das Abspielen der Tonfolge mittels des Lautsprechers 25 der tragbaren Authentifizierungsvorrichtung 20 informiert bzw. gewarnt wird, dass gerade ein Man-In-The-Middle-Angriff stattfindet.

Bei einer bevorzugten Ausführungsform ist in der Speichervorrichtung 13 der medizinischen Vorrichtung 10 eine individuelle Identifikationsnummer gespeichert, die die medizinische Vorrichtung 10 eindeutig identifiziert bzw. unterschiedlich zu Identifikationsnummern ist, die in Speichervorrichtungen von anderen der Vielzahl von medizinischen Vorrichtungen gespeichert sind. In diesem Fall kann in dem Signal, das mittels der Kommunikationsvorrichtung 12 ausgegeben wird und das Signal enthält, welches dem akustischen Signal entspricht, ferner ein Signal enthalten sein, welches der in der Speichervorrichtung 13 gespeicherten individuellen Identifikationsnummer entspricht. Die tragbare Authentifizierungsvorrichtung 20 kann dann nach Empfang des von der medizinischen Vorrichtung 10 ausgesendeten Signals auf der Eingabe-/Ausgabeeinrichtung 24, insbesondere auf deren Display, die Identifikationsnummer der medizinischen Vorrichtung 10 ausgeben, wodurch der Benutzer die medizinische Vorrichtung 10 identifizieren und gegebenenfalls einen Zugriff auf die medizinische Vorrichtung 10 durch einen unbefugten Benutzer verhindern kann.

Bei einer bevorzugten Ausführungsform ist die vorbestimmte Tonfolge durch die Identifikationsnummer der medizinischen Vorrichtung 10 derart bestimmt, dass unterschiedliche medizinische Vorrichtungen 10 unterschiedliche Tonfolgen ausgeben. Auf diese Weise kann der Benutzer vorteilhaft schnell erkennen, an welcher medizinischen Vorrichtung 10 er sich authentifiziert.

Gemäß einer weiteren Ausführungsform ist die tragbare Authentifizierungsvorrichtung 20 dazu eingerichtet, bei erfolgreich hergestellter drahtloser Kommunikationsverbindung zwischen der medizinischen Vorrichtung 10 und der tragbaren Authentifizierungsvorrichtung 20 drahtlos ein Signal, insbesondere Datensignal, mittels der Kommunikationseinrichtung 22 auszusenden, das ein Signal enthält, welches einem akustischen Signal, beispielsweise einer vorgegebenen oder zufälligen Tonfolge, entspricht. Dabei ist die medizinische Vorrichtung 10, insbesondere deren Kommunikationsvorrichtung 12, dazu eingerichtet, das Signal, das das Signal enthält, welches dem akustischen Signal entspricht, zu empfangen, basierend auf dem empfangenen Signal mittels der Steuervorrichtung 11 ein akustisches Signal zu erzeugen, das dem Signal, welches dem akustischen Signal entspricht, entspricht, und das erzeugte akustische Signal mittels des Lautsprechers 17 an die tragbare Authentifizierungsvorrichtung 20 auszugeben. Die tragbare Authentifizierungsvorrichtung 20 ist dazu eingerichtet, das akustische Signal mittels des Mikrophons 26 zu empfangen, und bei Empfang des akustischen Signals drahtlos ein Signal, insbesondere Datensignal, mittels der Kommunikationseinrichtung 22 an die medizinische Vorrichtung 10 auszusenden, das angibt, dass die tragbare Authentifizierungsvorrichtung 20 das akustische Signal empfangen hat. Die medizinische Vorrichtung 10, insbesondere deren Steuervorrichtung 11, ist dazu eingerichtet, in Abhängigkeit davon, ob sie das Signal, das angibt, dass die tragbare Authentifizierungsvorrichtung 20 das akustische Signal empfangen hat, mittels der Kommunikationsvorrichtung 12 empfängt oder nicht, zu bestimmen, ob sich die tragbare Authentifizierungsvorrichtung 20 an einer Position, an der eine akustische Kommunikation zwischen der medizinischen Vorrichtung 10 und der tragbaren Authentifizierungsvorrichtung 20 möglich ist, befindet oder nicht.

Da das von der medizinischen Vorrichtung 10 ausgesendete akustische Signal nur von der tragbaren Authentifizierungsvorrichtung 20 empfangbar ist, wenn diese sich an einer Position, an der eine akustische Kommunikation zwischen der medizinischen Vorrichtung 10 und der tragbaren Authentifizierungsvorrichtung 20 möglich ist, befindet, bestimmt die medizinische Vorrichtung 10 anhand des Empfangs des Signals, das angibt, dass die tragbare Authentifizierungsvorrichtung 20 das akustische Signal empfangen hat, dass sich die tragbare Authentifizierungsvorrichtung 20 an einer Position, an der eine akustische Kommunikation zwischen der medizinischen Vorrichtung 10 und der tragbaren Authentifizierungsvorrichtung 20 möglich ist, befindet.

Falls die medizinische Vorrichtung 10 nach Ablauf einer vorgegebenen Zeitdauer nach Aussendung bzw. Ausgabe des akustischen Signals von der tragbaren Authentifizierungsvorrichtung 20 hingegen kein Signal empfängt, das angibt, dass die tragbare Authentifizierungsvorrichtung 20 das akustische Signal empfangen hat, bestimmt die medizinische Vorrichtung 10, dass sich die tragbare Authentifizierungsvorrichtung 20 nicht an einer Position, an der eine akustische Kommunikation zwischen der medizinischen Vorrichtung 10 und der tragbaren Authentifizierungsvorrichtung 20 möglich ist, befindet.

Gemäß einer weiteren anderen Ausführungsform ist die tragbare Authentifizierungsvorrichtung 20 dazu eingerichtet, bei erfolgreich hergestellter drahtloser Kommunikationsverbindung zwischen der medizinischen Vorrichtung 10 und der tragbaren Authentifizierungsvorrichtung 20 ein vorgegebenes akustisches Signal, vorzugsweise eine vorgegebene Tonfolge, mittels des Lautsprechers 25 auszugeben. Dabei ist die medizinische Vorrichtung 10 dazu eingerichtet, in Abhängigkeit davon, ob sie das vorgegebene akustische Signal empfängt oder nicht, zu bestimmen, ob sich die tragbare Authentifizierungsvorrichtung 20 an einer Position, an der eine akustische Kommunikation zwischen der medizinischen Vorrichtung 10 und der tragbaren Authentifizierungsvorrichtung 20 möglich ist, befindet oder nicht.

Da das von der tragbaren Authentifizierungsvorrichtung 20 ausgegebene vorgegebene akustische Signal nur von der medizinischen Vorrichtung 10 empfangbar ist, wenn sich die tragbare Authentifizierungsvorrichtung 20 an einer Position, an der eine akustische Kommunikation zwischen der medizinischen Vorrichtung 10 und der tragbaren Authentifizierungsvorrichtung 20 möglich ist, befindet, bestimmt die medizinische Vorrichtung 10 anhand des Empfangs des vorgegebenen akustischen Signals, dass sich die tragbare Authentifizierungsvorrichtung 20 an einer Position, an der eine akustische Kommunikation zwischen der medizinischen Vorrichtung 10 und der tragbaren Authentifizierungsvorrichtung 20 möglich ist, befindet.

Falls die medizinische Vorrichtung 10 nach Ablauf einer vorgegebenen Zeitdauer nach erfolgreich hergestellter drahtloser Kommunikationsverbindung zwischen der medizinischen Vorrichtung 10 und der tragbaren Authentifizierungsvorrichtung 20 hingegen das vorgegebene akustische Signal nicht empfängt, bestimmt die medizinische Vorrichtung 10, dass sich die tragbare Authentifizierungsvorrichtung 20 nicht an einer Position, an der eine akustische Kommunikation zwischen der medizinischen Vorrichtung 10 und der tragbaren Authentifizierungsvorrichtung 20 möglich ist, befindet.

Wenn die medizinische Vorrichtung 10 feststellt, dass der Benutzer, dem das entsprechende Authentifizierungssignal zugeordnet ist, für die Bedienung der spezifischen medizinischen Vorrichtung 10 zugelassen ist, und ferner bestimmt, dass sich die tragbare Authentifizierungsvorrichtung 20 an einer Position, an der eine akustische Kommunikation zwischen der medizinischen Vorrichtung 10 und der tragbaren Authentifizierungsvorrichtung 20 möglich ist, befindet, bestimmt die medizinische Vorrichtung 10, dass die Authentifizierung erfolgreich war, und gibt einen Zugriff auf die medizinische Vorrichtung 10 im Rahmen der dem Authentifizierungssignal in der Berechtigungsdatenbank zugeordneten Berechtigungen frei.

Wenn hingegen die medizinische Vorrichtung 10 feststellt, dass der Benutzer, dem das entsprechende Authentifizierungssignal zugeordnet ist, für die Bedienung der spezifischen medizinischen Vorrichtung 10 nicht zugelassen ist, oder bestimmt, dass sich die tragbare Authentifizierungsvorrichtung 20 nicht an einer Position, an der eine akustische Kommunikation zwischen der medizinischen Vorrichtung 10 und der tragbaren Authentifizierungsvorrichtung 20 möglich ist, befindet, bestimmt die medizinische Vorrichtung 10, dass die Authentifizierung nicht erfolgreich war, und blockiert einen Zugriff auf die medizinische Vorrichtung 10.

Gemäß einer bevorzugten Ausführungsform sind in der Speichervorrichtung 13 der medizinischen Vorrichtung 10 Patientendaten und/oder den Patientendaten zugeordnete Betriebsparameter der medizinischen Vorrichtung 10, die während einer Behandlung des Patienten mittels der medizinischen Vorrichtung 10 aufgezeichnet wurden, speicherbar oder gespeichert, wobei das Display der Eingabe-/Ausgabevorrichtung 14 zur Anzeige der Patientendaten und/oder der den Patientendaten zugeordneten Betriebsparameter der medizinischen Vorrichtung 10 eingerichtet ist. In diesem Fall ist die medizinische Vorrichtung 10 ferner derart eingerichtet, dass ein Benutzer mittels einer Eingabe unter Verwendung der Eingabe-/Ausgabevorrichtung 14 nur bewirken kann, dass die in der Speichervorrichtung 13 der medizinischen Vorrichtung 10 gespeicherten Patientendaten und/oder die den Patientendaten zugeordneten Betriebsparameter der medizinischen Vorrichtung 10, die in der Speichervorrichtung 13 gespeichert sind, auf dem Display der Eingabe-/Ausgabevorrichtung 14 angezeigt werden, wenn die medizinische Vorrichtung 10 bestimmt, dass die Authentifizierung erfolgreich ist.

Somit kann gemäß dieser bevorzugten Ausführungsform ein unbefugter Zugriff auf die in der Speichervorrichtung 13 der medizinischen Vorrichtung 10 gespeicherten Patientendaten wie etwa Name, Alter, Patientenidentifikationsnummer, Patientenhistorie, und Ähnliches, sowie ein unbefugter Zugriff auf während einer Behandlung des Patienten aufgezeichnete und in der Speichervorrichtung 13 der medizinischen Vorrichtung 10 abgespeicherte, dem Patienten zugeordnete Betriebsparameter der medizinischen Vorrichtung 10 verhindert oder zumindest erschwert werden.

Bei einer besonders bevorzugten Ausführungsform können die Patientendaten und/oder die den Patientendaten zugeordneten Betriebsparameter, die in der Speichervorrichtung 13 der medizinischen Vorrichtung 10 gespeichert sind, von einem Benutzer mittels der drahtlosen Kommunikationsverbindung zwischen der medizinischen Vorrichtung 10 und der tragbaren Authentifizierungsvorrichtung 20 nur in die Speichereinrichtung 23 der tragbaren Authentifizierungsvorrichtung 20 übertragen werden, wenn die medizinische Vorrichtung 10 bestimmt, dass die Authentifizierung erfolgreich ist.

Zusammenfassend können durch den bevorzugten zweikanaligen Authentifizierungsprozess die funktionale Sicherheit des Behandlungssystems und insbesondere der medizinischen Vorrichtung 10 sichergestellt werden und Man-In-The-Middle-Angriffe erschwert werden. Zudem wird der Benutzer durch Ausgabe des akustischen Signals durch den Lautsprecher 17 der medizinischen Vorrichtung 10 oder den Lautsprecher 25 der tragbaren Authentifizierungsvorrichtung 20 zusätzlich über einen stattfindenden Authentifizierungsvorgang informiert.

## Patentansprüche

1. Behandlungssystem (100) zur Behandlung eines Patienten, umfassend:
eine medizinische Vorrichtung (10), und
eine tragbare Authentifizierungsvorrichtung (20), wobei
die medizinische Vorrichtung (10) und die tragbare Authentifizierungsvorrichtung (20) dazu eingerichtet sind, drahtlos miteinander zu kommunizieren,
die medizinische Vorrichtung (10) dazu eingerichtet ist, bei erfolgreich hergestellter drahtloser Kommunikationsverbindung zwischen der medizinischen Vorrichtung (10) und der tragbaren Authentifizierungsvorrichtung (20) ein akustisches Signal auszugeben,
die tragbare Authentifizierungsvorrichtung (20) dazu eingerichtet ist, das akustische Signal zu empfangen, basierend auf dem empfangenen akustischen Signal ein Signal zu erzeugen, das ein Signal enthält, welches dem empfangenen akustischen Signal entspricht, und das erzeugte Signal drahtlos an die medizinische Vorrichtung (10) auszusenden, und
die medizinische Vorrichtung (10) dazu eingerichtet ist, in Abhängigkeit davon, ob sie das Signal, das das Signal enthält, welches dem von der tragbaren Authentifizierungsvorrichtung (20) empfangenen akustischen Signal entspricht, empfängt oder nicht, zu bestimmen, ob sich die tragbare Authentifizierungsvorrichtung (20) an einer Position, an der eine akustische Kommunikation zwischen der medizinischen Vorrichtung (10) und der tragbaren Authentifizierungsvorrichtung (20) möglich ist, befindet oder nicht, wobei die medizinische Vorrichtung (10) dazu eingerichtet ist, ein Abfragesignal zur Abfrage von Authentifizierungsinformationen drahtlos an die tragbare Authentifizierungsvorrichtung (20) auszusenden und ein Authentifizierungssignal drahtlos zu empfangen,
die tragbare Authentifizierungsvorrichtung (20) dazu eingerichtet ist zyklisch und/oder nach Empfang des Abfragesignals ein Authentifizierungssignal auszusenden, welches Informationen enthält, welche die tragbare Authentifizierungsvorrichtung (20) identifizieren,
die medizinische Vorrichtung (10) dazu eingerichtet ist, bei Empfang des Authentifizierungssignals von der tragbaren Authentifizierungsvorrichtung (20) eine Authentifikation durchzuführen, bei der geprüft wird, ob das Authentifizierungssignal einem in einer Berechtigungsdatenbank registrierten Benutzer oder einer in der Berechtigungsdatenbank registrierten tragbaren Authentifizierungsvorrichtung (20) zugeordnet ist oder nicht, und ob sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet oder nicht, und
zu bestimmen, dass die Authentifizierung erfolgreich ist, wenn das Authentifizierungssignal einem in der Berechtigungsdatenbank registrierten Benutzer oder einer in der Berechtigungsdatenbank registrierten tragbaren Authentifizierungsvorrichtung (20) zugeordnet ist und sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet.

2. Behandlungssystem (100) zur Behandlung eines Patienten, umfassend:
eine medizinische Vorrichtung (10), und
eine tragbare Authentifizierungsvorrichtung (20), wobei
die medizinische Vorrichtung (10) und die tragbare Authentifizierungsvorrichtung (20) dazu eingerichtet sind, drahtlos miteinander zu kommunizieren,
die medizinische Vorrichtung (10) dazu eingerichtet ist, bei erfolgreich hergestellter drahtloser Kommunikationsverbindung zwischen der medizinischen Vorrichtung (10) und der tragbaren Authentifizierungsvorrichtung (20) drahtlos ein Signal auszusenden, das ein Signal enthält, welches einem akustischen Signal entspricht,
die tragbare Authentifizierungsvorrichtung (20) dazu eingerichtet ist, das Signal, das das Signal enthält, welches dem akustischen Signal entspricht, zu empfangen, basierend auf dem empfangenen Signal ein akustisches Signal zu erzeugen, das dem Signal, welches dem akustischen Signal entspricht, entspricht, und das erzeugte akustische Signal an die medizinische Vorrichtung (10) auszugeben, und
die medizinische Vorrichtung (10) dazu eingerichtet ist, in Abhängigkeit davon, ob sie das akustische Signal empfängt oder nicht, zu bestimmen, ob sich die tragbare Authentifizierungsvorrichtung (20) an einer Position, an der eine akustische Kommunikation zwischen der medizinischen Vorrichtung (10) und der tragbaren Authentifizierungsvorrichtung (20) möglich ist, befindet oder nicht, wobei
die medizinische Vorrichtung (10) dazu eingerichtet ist, ein Abfragesignal zur Abfrage von Authentifizierungsinformationen drahtlos an die tragbare Authentifizierungsvorrichtung (20) auszusenden und ein Authentifizierungssignal drahtlos zu empfangen,
die tragbare Authentifizierungsvorrichtung (20) dazu eingerichtet ist zyklisch und/oder nach Empfang des Abfragesignals ein Authentifizierungssignal auszusenden, welches Informationen enthält, welche die tragbare Authentifizierungsvorrichtung (20) identifizieren,
die medizinische Vorrichtung (10) dazu eingerichtet ist, bei Empfang des Authentifizierungssignals von der tragbaren Authentifizierungsvorrichtung (20) eine Authentifikation durchzuführen, bei der geprüft wird, ob das Authentifizierungssignal einem in einer Berechtigungsdatenbank registrierten Benutzer oder einer in der Berechtigungsdatenbank registrierten tragbaren Authentifizierungsvorrichtung (20) zugeordnet ist oder nicht, und ob sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet oder nicht, und zu bestimmen, dass die Authentifizierung erfolgreich ist, wenn das Authentifizierungssignal einem in der Berechtigungsdatenbank registrierten Benutzer oder einer in der Berechtigungsdatenbank registrierten tragbaren Authentifizierungsvorrichtung (20) zugeordnet ist und sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet.

3. Behandlungssystem (100) zur Behandlung eines Patienten, umfassend:
eine medizinische Vorrichtung (10), und
eine tragbare Authentifizierungsvorrichtung (20), wobei
die medizinische Vorrichtung (10) und die tragbare Authentifizierungsvorrichtung (20) dazu eingerichtet sind, drahtlos miteinander zu kommunizieren,
die tragbare Authentifizierungsvorrichtung (20) dazu eingerichtet ist, bei erfolgreich hergestellter drahtloser Kommunikationsverbindung zwischen der medizinischen Vorrichtung (10) und der tragbaren Authentifizierungsvorrichtung (20) drahtlos ein Signal auszusenden, das ein Signal enthält, welches einem akustischen Signal entspricht,
die medizinische Vorrichtung (10) dazu eingerichtet ist, das Signal, das das Signal enthält, welches dem akustischen Signal entspricht, zu empfangen, basierend auf dem empfangenen Signal ein akustisches Signal zu erzeugen, das dem Signal, welches dem akustischen Signal entspricht, entspricht, und das erzeugte akustische Signal an die tragbare Authentifizierungsvorrichtung (20) auszugeben,
die tragbare Authentifizierungsvorrichtung (20) dazu eingerichtet ist, das akustische Signal zu empfangen, und bei Empfang des akustischen Signals drahtlos ein Signal an die medizinische Vorrichtung (10) auszusenden, das angibt, dass die tragbare Authentifizierungsvorrichtung (20) das akustische Signal empfangen hat, und
die medizinische Vorrichtung (10) dazu eingerichtet ist, in Abhängigkeit davon, ob sie das Signal, das angibt, dass die tragbare Authentifizierungsvorrichtung (20) das akustische Signal empfangen hat, empfängt oder nicht, zu bestimmen, ob sich die tragbare Authentifizierungsvorrichtung (20) an einer Position, an der eine akustische Kommunikation zwischen der medizinischen Vorrichtung (10) und der tragbaren Authentifizierungsvorrichtung (20) möglich ist, befindet oder nicht, wobei
die medizinische Vorrichtung (10) dazu eingerichtet ist, ein Abfragesignal zur Abfrage von Authentifizierungsinformationen drahtlos an die tragbare Authentifizierungsvorrichtung (20) auszusenden und ein Authentifizierungssignal drahtlos zu empfangen,
die tragbare Authentifizierungsvorrichtung (20) dazu eingerichtet ist zyklisch und/oder nach Empfang des Abfragesignals ein Authentifizierungssignal auszusenden, welches Informationen enthält, welche die tragbare Authentifizierungsvorrichtung (20) identifizieren,
die medizinische Vorrichtung (10) dazu eingerichtet ist, bei Empfang des Authentifizierungssignals von der tragbaren Authentifizierungsvorrichtung (20) eine Authentifikation durchzuführen, bei der geprüft wird, ob das Authentifizierungssignal einem in einer Berechtigungsdatenbank registrierten Benutzer oder einer in der Berechtigungsdatenbank registrierten tragbaren Authentifizierungsvorrichtung (20) zugeordnet ist oder nicht, und ob sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet oder nicht, und zu bestimmen, dass die Authentifizierung erfolgreich ist, wenn das Authentifizierungssignal einem in der Berechtigungsdatenbank registrierten Benutzer oder einer in der Berechtigungsdatenbank registrierten tragbaren Authentifizierungsvorrichtung (20) zugeordnet ist und sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet.

4. Behandlungssystem (100) zur Behandlung eines Patienten, umfassend:
eine medizinische Vorrichtung (10), und
eine tragbare Authentifizierungsvorrichtung (20), wobei
die medizinische Vorrichtung (10) und die tragbare Authentifizierungsvorrichtung (20) dazu eingerichtet sind, drahtlos miteinander zu kommunizieren,
die tragbare Authentifizierungsvorrichtung (20) dazu eingerichtet ist, bei erfolgreich hergestellter drahtloser Kommunikationsverbindung zwischen der medizinischen Vorrichtung (10) und der tragbaren Authentifizierungsvorrichtung (20) ein vorgegebenes akustisches Signal, vorzugsweise eine vorgegebene Tonfolge, auszugeben, und
die medizinische Vorrichtung (10) dazu eingerichtet ist, in Abhängigkeit davon, ob sie das vorgegebene akustische Signal empfängt oder nicht, zu bestimmen, ob sich die tragbare Authentifizierungsvorrichtung (20) an einer Position, an der eine akustische Kommunikation zwischen der medizinischen Vorrichtung (10) und der tragbaren Authentifizierungsvorrichtung (20) möglich ist, befindet oder nicht, wobei die medizinische Vorrichtung (10) dazu eingerichtet ist, ein Abfragesignal zur Abfrage von Authentifizierungsinformationen drahtlos an die tragbare Authentifizierungsvorrichtung (20) auszusenden und ein Authentifizierungssignal drahtlos zu empfangen,
die tragbare Authentifizierungsvorrichtung (20) dazu eingerichtet ist zyklisch und/oder nach Empfang des Abfragesignals ein Authentifizierungssignal auszusenden, welches Informationen enthält, welche die tragbare Authentifizierungsvorrichtung (20) identifizieren,
die medizinische Vorrichtung (10) dazu eingerichtet ist, bei Empfang des Authentifizierungssignals von der tragbaren Authentifizierungsvorrichtung (20) eine Authentifikation durchzuführen, bei der geprüft wird, ob das Authentifizierungssignal einem in einer Berechtigungsdatenbank registrierten Benutzer oder einer in der Berechtigungsdatenbank registrierten tragbaren Authentifizierungsvorrichtung (20) zugeordnet ist oder nicht, und ob sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet oder nicht, und zu bestimmen, dass die Authentifizierung erfolgreich ist, wenn das Authentifizierungssignal einem in der Berechtigungsdatenbank registrierten Benutzer oder einer in der Berechtigungsdatenbank registrierten tragbaren Authentifizierungsvorrichtung (20) zugeordnet ist und sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet.

5. Behandlungssystem (100) gemäß einem der Ansprüche 1 bis 3, wobei das akustische Signal eine zufällige oder eine vorgegebene Tonfolge ist, und das Signal, das das Signal enthält, welches dem akustischen Signal entspricht, ein Signal enthält, das der zufälligen oder der vorgegebenen Tonfolge entspricht.

6. Behandlungssystem (100) gemäß Anspruch 1 oder 2, wobei das akustische Signal eine vorgegebene Tonfolge ist, das Signal, das das Signal enthält, welches dem akustischen Signal entspricht, ein Signal enthält, das der vorgegebenen Tonfolge entspricht, und die Tonfolge durch eine individuelle Identifikationsnummer, die die medizinische Vorrichtung (10) eindeutig identifiziert, bestimmt ist.

7. Behandlungssystem (100) gemäß einem der vorhergehenden Ansprüche, wobei die medizinische Vorrichtung (10) und die tragbare Authentifizierungsvorrichtung (20) dazu eingerichtet sind, drahtlos über einen gemeinsamen Funkstandard miteinander zu kommunizieren und/oder die medizinische Vorrichtung (10) und die tragbare Authentifizierungsvorrichtung (20) dazu eingerichtet sind, drahtlos über ein Infrarotsignal miteinander zu kommunizieren.

8. Behandlungssystem (100) gemäß einem der Ansprüche 1 bis 7, wobei die medizinische Vorrichtung (10) ferner
eine Speichervorrichtung (13), in der Patientendaten und/oder den Patientendaten zugeordnete Betriebsparameter der medizinischen Vorrichtung (10), die während einer Behandlung des Patienten aufgezeichnet wurden, speicherbar sind, und
eine Eingabe-/Ausgabevorrichtung (14) mit einem Display zur Anzeige der Patientendaten und/oder der den Patientendaten zugeordneten Betriebsparameter der medizinischen Vorrichtung (10) aufweist, wobei
ein Benutzer mittels einer Eingabe unter Verwendung der Eingabe-/Ausgabevorrichtung (14) nur bewirken kann, dass die in der Speichervorrichtung (13) gespeicherten Patientendaten und/oder die den Patientendaten zugeordneten Betriebsparameter der medizinischen Vorrichtung (10), die in der Speichervorrichtung (13) gespeichert sind, auf dem Display der Eingabe-/Ausgabevorrichtung (14) angezeigt werden, wenn die medizinische Vorrichtung (10) bestimmt, dass die Authentifizierung erfolgreich ist.

9. Behandlungssystem (100) gemäß Anspruch 2, wobei die medizinische Vorrichtung (10) eine Speichervorrichtung (13) aufweist, in der eine individuelle Identifikationsnummer gespeichert ist, die die medizinische Vorrichtung (10) eindeutig identifiziert,
die medizinische Vorrichtung (10) dazu eingerichtet ist, in dem Signal, das ein Signal enthält, welches dem akustischen Signal entspricht, ferner ein Signal auszusenden, welches der in der Speichervorrichtung (13) gespeicherten individuellen Identifikationsnummer entspricht, und
die tragbare Authentifizierungsvorrichtung (20) eine Eingabe-/Ausgabevorrichtung (24) mit einem Display aufweist und dazu eingerichtet ist, nach Empfang des von der medizinischen Vorrichtung (10) ausgesendeten Signals die individuelle Identifikationsnummer auf dem Display auszugeben.

10. Behandlungssystem (100) gemäß Anspruch 9, wobei
in der Speichervorrichtung (13) Patientendaten und/oder den Patientendaten zugeordnete Betriebsparameter der medizinischen Vorrichtung (10), die während einer Behandlung des Patienten aufgezeichnet wurden, speicherbar sind, und
die medizinische Vorrichtung (10) ferner eine Eingabe-/Ausgabevorrichtung (14) mit einem Display zur Anzeige der Patientendaten und/oder der den Patientendaten zugeordneten Betriebsparameter der medizinischen Vorrichtung (10) aufweist, wobei
ein Benutzer mittels einer Eingabe unter Verwendung der Eingabe-/Ausgabevorrichtung (14) nur bewirken kann, dass die in der Speichervorrichtung (13) gespeicherten Patientendaten und/oder die den Patientendaten zugeordneten Betriebsparameter der medizinischen Vorrichtung (10), die in der Speichervorrichtung (13) gespeichert sind, auf dem Display der Eingabe-/Ausgabevorrichtung (14) angezeigt werden, wenn die medizinische Vorrichtung (10) bestimmt, dass die Authentifizierung erfolgreich ist.

11. Behandlungssystem (100) gemäß einem der vorhergehenden Ansprüche, wobei die tragbare Authentifizierungsvorrichtung (20) als ein Mobiltelefon oder als ein tragbarer Computer ausgebildet ist und/oder die medizinische Vorrichtung (10) eine Dialysevorrichtung ist, welche zur Durchführung einer Dialysebehandlung eingerichtet ist.

12. Verfahren zur Überprüfung der Authentifikation einer tragbaren Authentifizierungsvorrichtung (20) für ein Behandlungssystem (100) gemäß Anspruch 1, umfassend:
Ausgeben eines akustischen Signals durch die medizinische Vorrichtung (10),
Empfangen des akustischen Signals durch die tragbare Authentifizierungsvorrichtung (20), basierend auf dem empfangenen akustischen Signal Erzeugen, durch die tragbare Authentifizierungsvorrichtung (20), eines Signals, das ein Signal enthält, welches dem empfangenen akustischen Signal entspricht, und drahtloses Aussenden des erzeugten Signals an die medizinische Vorrichtung (10) durch die tragbare Authentifizierungsvorrichtung (20), und
Bestimmen, durch die medizinische Vorrichtung (10), in Abhängigkeit davon, ob sie das Signal, das das Signal enthält, welches dem von der tragbaren Authentifizierungsvorrichtung (20) empfangenen akustischen Signal entspricht, empfängt oder nicht, ob sich die tragbare Authentifizierungsvorrichtung (20) an einer Position, an der eine akustische Kommunikation zwischen der medizinischen Vorrichtung (10) und der tragbaren Authentifizierungsvorrichtung (20) möglich ist, befindet oder nicht,
drahtloses Aussenden, durch die medizinische Vorrichtung (10), eines Abfragesignals zur Abfrage von Authentifizierungsinformationen an die tragbare Authentifizierungsvorrichtung (20),
zyklisches Aussenden und/oder Aussenden nach Empfang des Abfragesignals eines Authentifizierungssignals, welches Informationen enthält, welche die tragbare Authentifizierungsvorrichtung (20) identifizieren, durch die tragbare Authentifizierungsvorrichtung (20),
drahtloses Empfangen, durch die medizinische Vorrichtung (10), des Authentifizierungssignals von der tragbaren Authentifizierungsvorrichtung (20), und Durchführen, durch die medizinische Vorrichtung (10), einer Authentifikation, bei der geprüft wird, ob das Authentifizierungssignal einem in einer Berechtigungsdatenbank registrierten Benutzer oder einer in der Berechtigungsdatenbank registrierten tragbaren Authentifizierungsvorrichtung (20) zugeordnet ist oder nicht, und ob sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet oder nicht, und Bestimmen, durch die medizinische Vorrichtung (10), dass die Authentifizierung erfolgreich ist, wenn das Authentifizierungssignal einem in der Berechtigungsdatenbank registrierten Benutzer oder einer in der Berechtigungsdatenbank registrierten tragbaren Authentifizierungsvorrichtung (20) zugeordnet ist und sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet.

13. Verfahren zur Überprüfung der Authentifikation einer tragbaren Authentifizierungsvorrichtung (20) für ein Behandlungssystem (100) gemäß Anspruch 2, umfassend:
drahtloses Aussenden eines Signals, das ein Signal enthält, welches einem akustischen Signal entspricht, durch die medizinische Vorrichtung (10), Empfangen, durch die tragbare Authentifizierungsvorrichtung (20), des Signals,
welches dem akustischen Signal entspricht, basierend auf dem empfangenen Signal Erzeugen, durch die tragbare Authentifizierungsvorrichtung (20), eines akustischen Signals, das dem Signal, welches dem akustischen Signal entspricht, entspricht, und Ausgeben des erzeugten akustischen Signals an die medizinische Vorrichtung (10), und
Bestimmen, durch die medizinische Vorrichtung (10), in Abhängigkeit davon, ob sie das akustische Signal empfängt oder nicht, ob sich die tragbare Authentifizierungsvorrichtung (20) an einer Position, an der eine akustische Kommunikation zwischen der medizinischen Vorrichtung (10) und der tragbaren Authentifizierungsvorrichtung (20) möglich ist, befindet oder nicht, drahtloses Aussenden, durch die medizinische Vorrichtung (10), eines Abfragesignals zur Abfrage von Authentifizierungsinformationen an die tragbare Authentifizierungsvorrichtung (20),
zyklisches Aussenden und/oder Aussenden nach Empfang des Abfragesignals eines Authentifizierungssignals, welches Informationen enthält, welche die tragbare Authentifizierungsvorrichtung (20) identifizieren, durch die tragbare Authentifizierungsvorrichtung (20),
drahtloses Empfangen, durch die medizinische Vorrichtung (10), des Authentifizierungssignals von der tragbaren Authentifizierungsvorrichtung (20), und Durchführen, durch die medizinische Vorrichtung (10), einer Authentifikation, bei der geprüft wird, ob das Authentifizierungssignal einem in einer Berechtigungsdatenbank registrierten Benutzer oder einer in der Berechtigungsdatenbank registrierten tragbaren Authentifizierungsvorrichtung (20) zugeordnet ist oder nicht, und ob sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet oder nicht, und Bestimmen, durch die medizinische Vorrichtung (10), dass die Authentifizierung erfolgreich ist, wenn das Authentifizierungssignal einem in der Berechtigungsdatenbank registrierten Benutzer oder einer in der Berechtigungsdatenbank registrierten tragbaren Authentifizierungsvorrichtung (20) zugeordnet ist und sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet.

14. Verfahren zur Überprüfung der Authentifikation einer tragbaren Authentifizierungsvorrichtung (20) für ein Behandlungssystem (100) gemäß Anspruch 3, umfassend:
drahtloses Aussenden eines Signals, das ein Signal enthält, welches einem akustischen Signal entspricht, durch die tragbare Authentifizierungsvorrichtung (20),
Empfangen, durch die medizinische Vorrichtung (10), des Signals, das das Signal enthält, welches dem akustischen Signal entspricht, basierend auf dem empfangenen Signal Erzeugen, durch die medizinische Vorrichtung (10), eines akustischen Signals, das dem Signal, welches dem akustischen Signal entspricht, entspricht, und Ausgeben des erzeugten akustischen Signals an die tragbare Authentifizierungsvorrichtung (20) durch die medizinische Vorrichtung (10),
drahtloses Aussenden eines Signals, das angibt, dass die tragbare Authentifizierungsvorrichtung (20) das akustische Signal empfangen hat, durch die tragbare Authentifizierungsvorrichtung (20), an die medizinische Vorrichtung (10), wenn die tragbare Authentifizierungsvorrichtung (20) das akustische Signal von der medizinischen Vorrichtung (10) empfangen hat, und
Bestimmen, durch die medizinische Vorrichtung (10), in Abhängigkeit davon, ob sie das Signal, das angibt, dass die tragbare Authentifizierungsvorrichtung (20) das akustische Signal empfangen hat, empfängt oder nicht, ob sich die tragbare Authentifizierungsvorrichtung (20) an einer Position, an der eine akustische Kommunikation zwischen der medizinischen Vorrichtung (10) und der tragbaren Authentifizierungsvorrichtung (20) möglich ist, befindet oder nicht,
drahtloses Aussenden, durch die medizinische Vorrichtung (10), eines Abfragesignals zur Abfrage von Authentifizierungsinformationen an die tragbare Authentifizierungsvorrichtung (20),
zyklisches Aussenden und/oder Aussenden nach Empfang des Abfragesignals eines Authentifizierungssignals, welches Informationen enthält, welche die tragbare Authentifizierungsvorrichtung (20) identifizieren, durch die tragbare Authentifizierungsvorrichtung (20),
drahtloses Empfangen, durch die medizinische Vorrichtung (10), des Authentifizierungssignals von der tragbaren Authentifizierungsvorrichtung (20), und Durchführen, durch die medizinische Vorrichtung (10), einer Authentifikation, bei der geprüft wird, ob das Authentifizierungssignal einem in einer Berechtigungsdatenbank registrierten Benutzer oder einer in der Berechtigungsdatenbank registrierten tragbaren Authentifizierungsvorrichtung (20) zugeordnet ist oder nicht, und ob sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet oder nicht, und Bestimmen, durch die medizinische Vorrichtung (10), dass die Authentifizierung erfolgreich ist, wenn das Authentifizierungssignal einem in der Berechtigungsdatenbank registrierten Benutzer oder einer in der Berechtigungsdatenbank registrierten tragbaren Authentifizierungsvorrichtung (20) zugeordnet ist und sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet.

15. Verfahren zur Überprüfung der Authentifikation einer tragbaren Authentifizierungsvorrichtung (20) für ein Behandlungssystem (100) gemäß Anspruch 4, umfassend:
Ausgeben eines vorgegebenen akustischen Signals, vorzugsweise einer vorgegebenen Tonfolge, durch die tragbare Authentifizierungsvorrichtung (20), und
Bestimmen, durch die medizinische Vorrichtung (10), in Abhängigkeit davon, ob sie das vorgegebene akustische Signal empfängt oder nicht, ob sich die tragbare Authentifizierungsvorrichtung (20) an einer Position, an der eine akustische Kommunikation zwischen der medizinischen Vorrichtung (10) und der tragbaren Authentifizierungsvorrichtung (20) möglich ist, befindet oder nicht,
drahtloses Aussenden, durch die medizinische Vorrichtung (10), eines Abfragesignals zur Abfrage von Authentifizierungsinformationen an die tragbare Authentifizierungsvorrichtung (20),
zyklisches Aussenden und/oder Aussenden nach Empfang des Abfragesignals eines Authentifizierungssignals, welches Informationen enthält, welche die tragbare Authentifizierungsvorrichtung (20) identifizieren, durch die tragbare Authentifizierungsvorrichtung (20),
drahtloses Empfangen, durch die medizinische Vorrichtung (10), des Authentifizierungssignals von der tragbaren Authentifizierungsvorrichtung (20), und Durchführen, durch die medizinische Vorrichtung (10), einer Authentifikation, bei der geprüft wird, ob das Authentifizierungssignal einem in einer Berechtigungsdatenbank registrierten Benutzer oder einer in der Berechtigungsdatenbank registrierten tragbaren Authentifizierungsvorrichtung (20) zugeordnet ist oder nicht, und ob sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet oder nicht, und Bestimmen, durch die medizinische Vorrichtung (10), dass die Authentifizierung erfolgreich ist, wenn das Authentifizierungssignal einem in der Berechtigungsdatenbank registrierten Benutzer oder einer in der Berechtigungsdatenbank registrierten tragbaren Authentifizierungsvorrichtung (20) zugeordnet ist und sich die tragbare Authentifizierungsvorrichtung (20) an der Position, an der die akustische Kommunikation zwischen der medizinischen Vorrichtung (10) und der tragbaren Authentifizierungsvorrichtung (20) möglich ist, befindet.

## Claims

1. Treatment system (100) for treating a patient, comprising:
a medical device (10), and
a portable authentication device (20), wherein
the medical device (10) and the portable authentication device (20) are configured to communicate wirelessly with each other,
the medical device (10) is configured to output an acoustic signal when a wireless communication connection is successfully established between the medical device (10) and the portable authentication device (20),
the portable authentication device (20) is configured to receive the acoustic signal, to generate, based on the received acoustic signal, a signal containing a signal corresponding to the received acoustic signal, and to wirelessly transmit the generated signal to the medical device (10), and
the medical device (10) is configured to determine, depending on whether or not it receives the signal containing the signal corresponding to the acoustic signal received by the portable authentication device (20), whether the portable authentication device (20) is located at a position where acoustic communication between the medical device (10) and the portable authentication device (20) is possible, wherein
the medical device (10) is configured to wirelessly transmit a query signal to query authentication information to the portable authentication device (20) and to wirelessly receive an authentication signal,
the portable authentication device (20) is configured to cyclically and/or upon receipt of the query signal transmit an authentication signal containing information identifying the portable authentication device (20),
the medical device (10) is configured, upon receipt of the authentication signal from the portable authentication device (20), to perform authentication by checking whether the authentication signal is associated with a user registered in an authorization database or with a portable authentication device (20) registered in the authorization database, and whether the portable authentication device (20) is located at the position where acoustic communication between the medical device (10) and the portable authentication device (20) is possible, and to determine that the authentication is successful if the authentication signal is associated with a user registered in the authorization database or with a portable authentication device (20) registered in the authorization database and the portable authentication device (20) is located at the position where acoustic communication between the medical device (10) and the portable authentication device (20) is possible.

2. Treatment system (100) for treating a patient, comprising:
a medical device (10), and
a portable authentication device (20), wherein,
the medical device (10) and the portable authentication device (20) are configured to communicate wirelessly with each other,
the medical device (10) is configured, upon successful establishment of a wireless communication connection between the medical device (10) and the portable authentication device (20), to wirelessly transmit a signal containing a signal corresponding to an acoustic signal,
the portable authentication device (20) is configured to receive the signal containing the signal corresponding to the acoustic signal, to generate, based on the received signal, an acoustic signal corresponding to the signal corresponding to the acoustic signal, and to output the generated acoustic signal to the medical device (10), and
the medical device (10) is configured to determine, depending on whether or not it receives the acoustic signal, whether the portable authentication device (20) is located at a position where acoustic communication between the medical device (10) and the portable authentication device (20) is possible, wherein
the medical device (10) is configured to wirelessly transmit a query signal for querying authentication information to the portable authentication device (20) and to wirelessly receive an authentication signal,
the portable authentication device (20) is configured to cyclically and/or upon receipt of the query signal transmit an authentication signal containing information identifying the portable authentication device (20),
the medical device (10) is configured, upon receipt of the authentication signal from the portable authentication device (20), to perform authentication by checking whether the authentication signal is associated with a user registered in an authorization database or with a portable authentication device (20) registered in the authorization database, and whether the portable authentication device (20) is located at the position where acoustic communication between the medical device (10) and the portable authentication device (20) is possible, and to determine that the authentication is successful if the authentication signal is associated with a user registered in the authorization database or with a portable authentication device (20) registered in the authorization database and the portable authentication device (20) is located at the position where acoustic communication between the medical device (10) and the portable authentication device (20) is possible.

3. Treatment system (100) for treating a patient, comprising:
a medical device (10), and
a portable authentication device (20), wherein
the medical device (10) and the portable authentication device (20) are configured to communicate wirelessly with each other,
the portable authentication device (20) is configured, upon successful establishment of a wireless communication connection between the medical device (10) and the portable authentication device (20), to wirelessly transmit a signal containing a signal corresponding to an acoustic signal,
the medical device (10) is configured to receive the signal containing the signal corresponding to the acoustic signal, to generate, based on the received signal, an acoustic signal corresponding to the signal corresponding to the acoustic signal, and to output the generated acoustic signal to the portable authentication device (20),
the portable authentication device (20) is configured to receive the acoustic signal and, upon receipt of the acoustic signal, to wirelessly transmit to the medical device (10) a signal indicating that the portable authentication device (20) has received the acoustic signal, and
the medical device (10) is configured to determine, depending on whether or not it receives the signal indicating that the portable authentication device (20) has received the acoustic signal, whether the portable authentication device (20) is located at a position where acoustic communication between the medical device (10) and the portable authentication device (20) is possible, wherein
the medical device (10) is configured to wirelessly transmit a query signal for querying authentication information to the portable authentication device (20) and to wirelessly receive an authentication signal,
the portable authentication device (20) is configured to cyclically and/or upon receipt of the query signal transmit an authentication signal containing information identifying the portable authentication device (20),
the medical device (10) is configured, upon receipt of the authentication signal from the portable authentication device (20), to perform authentication by checking whether the authentication signal is associated with a user registered in an authorization database or with a portable authentication device (20) registered in the authorization database, and whether the portable authentication device (20) is located at the position where acoustic communication between the medical device (10) and the portable authentication device (20) is possible, and to determine that the authentication is successful if the authentication signal is associated with a user registered in the authorization database or with a portable authentication device (20) registered in the authorization database and the portable authentication device (20) is located at the position where acoustic communication between the medical device (10) and the portable authentication device (20) is possible.

4. Treatment system (100) for treating a patient, comprising:
a medical device (10), and
a portable authentication device (20), wherein
the medical device (10) and the portable authentication device (20) are configured to communicate wirelessly with each other,
the portable authentication device (20) is configured, upon successful establishment of a wireless communication connection between the medical device (10) and the portable authentication device (20), to output a predetermined acoustic signal, preferably a predetermined tone sequence, and
the medical device (10) is configured to determine, depending on whether or not it receives the predetermined acoustic signal, whether the portable authentication device (20) is located at a position where acoustic communication between the medical device (10) and the portable authentication device (20) is possible, wherein
the medical device (10) is configured to wirelessly transmit a query signal for querying authentication information to the portable authentication device (20) and to wirelessly receive an authentication signal,
the portable authentication device (20) is configured to cyclically and/or upon receipt of the query signal transmit an authentication signal containing information identifying the portable authentication device (20),
the medical device (10) is configured, upon receipt of the authentication signal from the portable authentication device (20), to perform authentication by checking whether the authentication signal is associated with a user registered in an authorization database or with a portable authentication device (20) registered in the authorization database, and whether the portable authentication device (20) is located at the position where acoustic communication between the medical device (10) and the portable authentication device (20) is possible, and to determine that the authentication is successful if the authentication signal is associated with a user registered in the authorization database or with a portable authentication device (20) registered in the authorization database and the portable authentication device (20) is located at the position where acoustic communication between the medical device (10) and the portable authentication device (20) is possible.

5. Treatment system (100) according to any one of claims 1 to 3, wherein the acoustic signal is a random or a predetermined tone sequence, and the signal containing the signal corresponding to the acoustic signal contains a signal corresponding to the random or the predetermined tone sequence.

6. Treatment system (100) according to claim 1 or 2, wherein the acoustic signal is a predetermined tone sequence, the signal containing the signal corresponding to the acoustic signal contains a signal corresponding to the predetermined tone sequence, and the tone sequence is determined by an individual identification number that uniquely identifies the medical device (10).

7. Treatment system (100) according to any one of the preceding claims, wherein the medical device (10) and the portable authentication device (20) are configured to communicate wirelessly with each other via a common radio standard, and/or the medical device (10) and the portable authentication device (20) are configured to communicate wirelessly with each other via an infrared signal.

8. Treatment system (100) according to any one of claims 1 to 7, wherein the medical device (10) further comprises:
a storage device (13) configured to store patient data and/or operating parameters of the medical device (10) associated with the patient data, which were recorded during treatment of the patient, and
an input/output device (14) with a display for displaying the patient data and/or the operating parameters of the medical device (10) associated with the patient data, wherein a user can only cause, by means of an input using the input/output device (14), the patient data stored in the storage device (13) and/or the operating parameters of the medical device (10) associated with the patient data, which are stored in the storage device (13), to be displayed on the display of the input/output device (14) if the medical device (10) determines that the authentication is successful.

9. Treatment system (100) according to claim 2, wherein the medical device (10) comprises a storage device (13) in which an individual identification number uniquely identifying the medical device (10) is stored,
the medical device (10) is configured to further transmit, in the signal containing a signal corresponding to the acoustic signal, a signal corresponding to the individual identification number stored in the storage device (13), and
the portable authentication device (20) comprises an input/output device (24) with a display and is configured to display the individual identification number on the display after receiving the signal transmitted by the medical device (10).

10. Treatment system (100) according to claim 9, wherein patient data and/or operating parameters of the medical device (10) associated with the patient data, which were recorded during treatment of the patient, are storable in the storage device (13), and
the medical device (10) further comprises an input/output device (14) with a display for displaying the patient data and/or the operating parameters of the medical device (10) associated with the patient data,
wherein a user can only cause, by means of an input using the input/output device (14), the patient data stored in the storage device (13) and/or the operating parameters of the medical device (10) associated with the patient data, which are stored in the storage device (13), to be displayed on the display of the input/output device (14) if the medical device (10) determines that the authentication is successful.

11. Treatment system (100) according to any one of the preceding claims, wherein the portable authentication device (20) is designed as a mobile telephone or as a portable computer, and/or the medical device (10) is a dialysis device configured to perform a dialysis treatment.

12. Method for verifying the authentication of a portable authentication device (20) for a treatment system (100) according to claim 1, comprising:
outputting an acoustic signal by the medical device (10),
receiving the acoustic signal by the portable authentication device (20), generating, by the portable authentication device (20) and based on the received acoustic signal, a signal containing a signal corresponding to the received acoustic signal, and wirelessly transmitting the generated signal to the medical device (10) by the portable authentication device (20), and
determining, by the medical device (10) and depending on whether it receives the signal containing the signal corresponding to the acoustic signal received by the portable authentication device (20), whether the portable authentication device (20) is located at a position where acoustic communication between the medical device (10) and the portable authentication device (20) is possible,
wirelessly transmitting, by the medical device (10), a query signal for querying authentication information to the portable authentication device (20),
cyclically transmitting and/or transmitting, by the portable authentication device (20) upon receipt of the query signal, an authentication signal containing information identifying the portable authentication device (20),
wirelessly receiving, by the medical device (10), the authentication signal from the portable authentication device (20), and performing, by the medical device (10), an authentication by checking whether the authentication signal is associated with a user registered in an authorization database or with a portable authentication device (20) registered in the authorization database, and whether the portable authentication device (20) is located at the position where acoustic communication between the medical device (10) and the portable authentication device (20) is possible, and determining, by the medical device (10), that the authentication is successful if the authentication signal is associated with a user registered in the authorization database or with a portable authentication device (20) registered in the authorization database and the portable authentication device (20) is located at the position where acoustic communication between the medical device (10) and the portable authentication device (20) is possible.

13. Method for verifying the authentication of a portable authentication device (20) for a treatment system (100) according to claim 2, comprising:
wirelessly transmitting, by the medical device (10), a signal containing a signal corresponding to an acoustic signal,
receiving, by the portable authentication device (20), the signal corresponding to the acoustic signal, generating, by the portable authentication device (20) and based on the received signal, an acoustic signal corresponding to the signal corresponding to the acoustic signal, and outputting the generated acoustic signal to the medical device (10), and
determining, by the medical device (10) and depending on whether it receives the acoustic signal, whether the portable authentication device (20) is located at a position where acoustic communication between the medical device (10) and the portable authentication device (20) is possible,
wirelessly transmitting, by the medical device (10), a query signal for querying authentication information to the portable authentication device (20),
cyclically transmitting and/or transmitting, by the portable authentication device (20) upon receipt of the query signal, an authentication signal containing information identifying the portable authentication device (20),
wirelessly receiving, by the medical device (10), the authentication signal from the portable authentication device (20), and performing, by the medical device (10), an authentication by checking whether the authentication signal is associated with a user registered in an authorization database or with a portable authentication device (20) registered in the authorization database, and whether the portable authentication device (20) is located at the position where acoustic communication between the medical device (10) and the portable authentication device (20) is possible, and determining, by the medical device (10), that the authentication is successful if the authentication signal is associated with a user registered in the authorization database or with a portable authentication device (20) registered in the authorization database and the portable authentication device (20) is located at the position where acoustic communication between the medical device (10) and the portable authentication device (20) is possible.

14. Method for verifying the authentication of a portable authentication device (20) for a treatment system (100) according to claim 3, comprising:
wirelessly transmitting, by the portable authentication device (20), a signal containing a signal corresponding to an acoustic signal,
receiving, by the medical device (10), the signal containing the signal corresponding to the acoustic signal, generating, by the medical device (10) and based on the received signal, an acoustic signal corresponding to the signal corresponding to the acoustic signal, and outputting the generated acoustic signal to the portable authentication device (20) by the medical device (10),
wirelessly transmitting, by the portable authentication device (20), a signal indicating that the portable authentication device (20) has received the acoustic signal to the medical device (10) when the portable authentication device (20) has received the acoustic signal from the medical device (10), and
determining, by the medical device (10) and depending on whether it receives the signal indicating that the portable authentication device (20) has received the acoustic signal, whether the portable authentication device (20) is located at a position where acoustic communication between the medical device (10) and the portable authentication device (20) is possible,
wirelessly transmitting, by the medical device (10), a query signal for querying authentication information to the portable authentication device (20),
cyclically transmitting and/or transmitting, by the portable authentication device (20) upon receipt of the query signal, an authentication signal containing information identifying the portable authentication device (20),
wirelessly receiving, by the medical device (10), the authentication signal from the portable authentication device (20), and performing, by the medical device (10), an authentication by checking whether the authentication signal is associated with a user registered in an authorization database or with a portable authentication device (20) registered in the authorization database, and whether the portable authentication device (20) is located at the position where acoustic communication between the medical device (10) and the portable authentication device (20) is possible, and
determining, by the medical device (10), that the authentication is successful if the authentication signal is associated with a user registered in the authorization database or with a portable authentication device (20) registered in the authorization database and the portable authentication device (20) is located at the position where acoustic communication between the medical device (10) and the portable authentication device (20) is possible.

15. Method for verifying the authentication of a portable authentication device (20) for a treatment system (100) according to claim 4, comprising:
outputting, by the portable authentication device (20), a predetermined acoustic signal, preferably a predetermined tone sequence, and
determining, by the medical device (10) and depending on whether it receives the predetermined acoustic signal, whether the portable authentication device (20) is located at a position where acoustic communication between the medical device (10) and the portable authentication device (20) is possible,
wirelessly transmitting, by the medical device (10), a query signal for querying authentication information to the portable authentication device (20),
cyclically transmitting and/or transmitting, by the portable authentication device (20) upon receipt of the query signal, an authentication signal containing information identifying the portable authentication device (20),
wirelessly receiving, by the medical device (10), the authentication signal from the portable authentication device (20), and performing, by the medical device (10), an authentication by checking whether the authentication signal is associated with a user registered in an authorization database or with a portable authentication device (20) registered in the authorization database, and whether the portable authentication device (20) is located at the position where acoustic communication between the medical device (10) and the portable authentication device (20) is possible, and
determining, by the medical device (10), that the authentication is successful if the authentication signal is associated with a user registered in the authorization database or with a portable authentication device (20) registered in the authorization database and the portable authentication device (20) is located at the position where acoustic communication between the medical device (10) and the portable authentication device (20) is possible.

## Revendications

1. Système de traitement (100) destiné à traiter un patient, comprenant :
un dispositif médical (10), et
un dispositif d'authentification portable (20), dans lequel
le dispositif médical (10) et le dispositif d'authentification portable (20) sont configurés pour communiquer ensemble sans fil ;
le dispositif médical (10) est configuré pour transmettre un signal acoustique dès lors qu'une liaison de communication sans fil a été établie avec succès entre le dispositif médical (10) et le dispositif d'authentification portable (20),
le dispositif d'authentification portable (20) est configuré pour recevoir le signal acoustique, produire, en se basant sur le signal acoustique reçu, un signal qui contient un signal qui correspond au signal acoustique reçu, et envoyer sans fil le signal produit au dispositif médical (10), et
le dispositif médical (10) est configuré pour, selon qu'il reçoit ou non le signal qui contient le signal qui correspond au signal acoustique reçu depuis le dispositif d'authentification portable (20), déterminer si le dispositif d'authentification portable (20) se trouve ou non dans une position où une communication acoustique entre le dispositif médical (10) et le dispositif d'authentification portable (20) est possible, dans lequel
le dispositif médical (10) est configuré pour envoyer sans fil un signal d'interrogation au dispositif d'authentification portable (20) pour l'interrogation d'informations d'authentification et pour recevoir sans fil un signal d'authentification,
le dispositif d'authentification portable (20) est configuré pour envoyer de façon cyclique et/ou après réception du signal d'interrogation un signal d'authentification qui contient des informations qui identifient le dispositif d'authentification portable (20),
le dispositif médical (10) est configuré pour réaliser, à réception du signal d'authentification depuis le dispositif d'authentification portable (20), une authentification par laquelle est vérifié si le signal d'authentification est attribué ou non à un utilisateur enregistré dans une base de données d'autorisation ou à un dispositif d'authentification portable (20) enregistré dans la base de données d'autorisation, et si le dispositif d'authentification portable (20) se trouve ou non dans la position où la communication acoustique entre le dispositif médical (10) et le dispositif d'authentification portable (20) est possible, et pour déterminer que l'authentification est réussie lorsque le signal d'authentification est attribué à un utilisateur enregistré dans la base de données d'autorisation ou à un dispositif d'authentification portable (20) enregistré dans la base de données d'autorisation, et le dispositif d'authentification portable (20) se trouve dans la position où la communication acoustique entre le dispositif médical (10) et le dispositif d'authentification portable (20) est possible.

2. Système de traitement (100) destiné à traiter un patient, comprenant :
un dispositif médical (10), et
un dispositif d'authentification portable (20), dans lequel
le dispositif médical (10) et le dispositif d'authentification portable (20) sont configurés pour communiquer ensemble sans fil ;
le dispositif médical (10) est configuré pour envoyer sans fil un signal qui correspond à un signal acoustique dès lors qu'une liaison de communication sans fil a été établie avec succès entre le dispositif médical (10) et le dispositif d'authentification portable (20),
le dispositif d'authentification portable (20) est configuré pour recevoir le signal qui contient le signal qui correspond au signal acoustique, produire, en se basant sur le signal reçu, un signal acoustique qui correspond au signal qui correspond au signal acoustique, et transmettre le signal acoustique produit au dispositif médical (10), et
le dispositif médical (10) est configuré pour, selon qu'il reçoit ou non le signal acoustique, déterminer si le dispositif d'authentification portable (20) se trouve ou non dans une position où une communication acoustique entre le dispositif médical (10) et le dispositif d'authentification portable (20) est possible, dans lequel
le dispositif médical (10) est configuré pour envoyer sans fil un signal d'interrogation au dispositif d'authentification portable (20) pour l'interrogation d'informations d'authentification et pour recevoir sans fil un signal d'authentification,
le dispositif d'authentification portable (20) est configuré pour envoyer de façon cyclique et/ou après réception du signal d'interrogation un signal d'authentification qui contient des informations qui identifient le dispositif d'authentification portable (20),
le dispositif médical (10) est configuré pour réaliser, à réception du signal d'authentification depuis le dispositif d'authentification portable (20), une authentification par laquelle est vérifié si le signal d'authentification est attribué ou non à un utilisateur enregistré dans une base de données d'autorisation ou à un dispositif d'authentification portable (20) enregistré dans la base de données d'autorisation, et si le dispositif d'authentification portable (20) se trouve ou non dans la position où la communication acoustique entre le dispositif médical (10) et le dispositif d'authentification portable (20) est possible, et pour déterminer que l'authentification est réussie lorsque le signal d'authentification est attribué à un utilisateur enregistré dans la base de données d'autorisation ou à un dispositif d'authentification portable (20) enregistré dans la base de données d'autorisation, et le dispositif d'authentification portable (20) se trouve dans la position où la communication acoustique entre le dispositif médical (10) et le dispositif d'authentification portable (20) est possible.

3. Système de traitement (100) destiné à traiter un patient, comprenant :
un dispositif médical (10), et
un dispositif d'authentification portable (20), dans lequel
le dispositif médical (10) et le dispositif d'authentification portable (20) sont configurés pour communiquer ensemble sans fil ;
le dispositif d'authentification portable (20) est configuré pour envoyer sans fil un signal qui contient un signal qui correspond à un signal acoustique dès lors qu'une liaison de communication sans fil a été établie avec succès entre le dispositif médical (10) et le dispositif d'authentification portable (20),
le dispositif médical (10) est configuré pour recevoir le signal qui contient le signal qui correspond au signal acoustique, produire, en se basant sur le signal reçu, un signal acoustique qui correspond au signal qui correspond au signal acoustique, et transmettre le signal acoustique produit au dispositif d'authentification portable (20),
le dispositif d'authentification portable (20) est configuré pour recevoir le signal acoustique, et à réception du signal acoustique pour envoyer sans fil au dispositif médical (10) un signal qui indique que le dispositif d'authentification portable (20) a reçu le signal acoustique, et
le dispositif médical (10) est configuré pour, selon qu'il reçoit ou non le signal qui indique que le dispositif d'authentification portable (20) a reçu le signal acoustique, déterminer si le dispositif d'authentification portable (20) se trouve ou non dans une position où une communication acoustique entre le dispositif médical (10) et le dispositif d'authentification portable (20) est possible, dans lequel
le dispositif médical (10) est configuré pour envoyer sans fil un signal d'interrogation au dispositif d'authentification portable (20) pour l'interrogation d'informations d'authentification et pour recevoir sans fil un signal d'authentification,
le dispositif d'authentification portable (20) est configuré pour envoyer de façon cyclique et/ou après réception du signal d'interrogation un signal d'authentification qui contient des informations qui identifient le dispositif d'authentification portable (20),
le dispositif médical (10) est configuré pour réaliser, à réception du signal d'authentification depuis le dispositif d'authentification portable (20), une authentification par laquelle est vérifié si le signal d'authentification est attribué ou non à un utilisateur enregistré dans une base de données d'autorisation ou à un dispositif d'authentification portable (20) enregistré dans la base de données d'autorisation, et si le dispositif d'authentification portable (20) se trouve ou non dans la position où la communication acoustique entre le dispositif médical (10) et le dispositif d'authentification portable (20) est possible, et pour déterminer que l'authentification est réussie lorsque le signal d'authentification est attribué à un utilisateur enregistré dans la base de données d'autorisation ou à un dispositif d'authentification portable (20) enregistré dans la base de données d'autorisation, et le dispositif d'authentification portable (20) se trouve dans la position où la communication acoustique entre le dispositif médical (10) et le dispositif d'authentification portable (20) est possible.

4. Système de traitement (100) destiné à traiter un patient, comprenant :
un dispositif médical (10), et
un dispositif d'authentification portable (20), dans lequel
le dispositif médical (10) et le dispositif d'authentification portable (20) sont configurés pour communiquer ensemble sans fil ;
le dispositif d'authentification portable (20) est configuré pour transmettre un signal acoustique prédéfini, de préférence une suite de notes prédéfinie, dès lors qu'une liaison de communication sans fil a été établie avec succès entre le dispositif médical (10) et le dispositif d'authentification portable (20), et
le dispositif médical (10) est configuré pour, selon qu'il reçoit ou non le signal acoustique prédéfini, déterminer si le dispositif d'authentification portable (20) se trouve ou non dans une position où une communication acoustique entre le dispositif médical (10) et le dispositif d'authentification portable (20) est possible, dans lequel
le dispositif médical (10) est configuré pour envoyer sans fil un signal d'interrogation au dispositif d'authentification portable (20) pour l'interrogation d'informations d'authentification et pour recevoir sans fil un signal d'authentification,
le dispositif d'authentification portable (20) est configuré pour envoyer de façon cyclique et/ou après réception du signal d'interrogation un signal d'authentification qui contient des informations qui identifient le dispositif d'authentification portable (20),
le dispositif médical (10) est configuré pour réaliser, à réception du signal d'authentification depuis le dispositif d'authentification portable (20), une authentification par laquelle est vérifié si le signal d'authentification est attribué ou non à un utilisateur enregistré dans une base de données d'autorisation ou à un dispositif d'authentification portable (20) enregistré dans la base de données d'autorisation, et si le dispositif d'authentification portable (20) se trouve ou non dans la position où la communication acoustique entre le dispositif médical (10) et le dispositif d'authentification portable (20) est possible, et pour déterminer que l'authentification est réussie lorsque le signal d'authentification est attribué à un utilisateur enregistré dans la base de données d'autorisation ou à un dispositif d'authentification portable (20) enregistré dans la base de données d'autorisation, et le dispositif d'authentification portable (20) se trouve dans la position où la communication acoustique entre le dispositif médical (10) et le dispositif d'authentification portable (20) est possible.

5. Système de traitement (100) selon l'une des revendications 1 à 3, dans lequel le signal acoustique est une suite de notes aléatoire ou prédéfinie, et le signal qui contient le signal qui correspond au signal acoustique contient un signal qui correspond à la suite de notes aléatoire ou prédéfinie.

6. Système de traitement (100) selon la revendication 1 ou 2, dans lequel le signal acoustique est une suite de notes prédéfinie, le signal qui contient le signal qui correspond au signal acoustique contient un signal qui correspond à la suite de notes prédéfinie, et la suite de notes est déterminée par un numéro d'identification individuel qui identifie clairement le dispositif médical (10).

7. Système de traitement (100) selon l'une des revendications précédentes, dans lequel le dispositif médical (10) et le dispositif d'authentification portable (20) sont configurés pour communiquer ensemble sans fil via une norme radio commune et/ou le dispositif médical (10) et le dispositif d'authentification portable (20) sont configurés pour communiquer ensemble sans fil via un signal infrarouge.

8. Système de traitement (100) selon l'une des revendications 1 à 7, dans lequel le dispositif médical (10) comprend en outre
un dispositif de stockage (13) dans lequel peuvent être stockés des données de patient et/ou des paramètres de fonctionnement attribués aux données de patient du dispositif médical (10), qui avaient été enregistrés pendant un traitement du patient, et
un dispositif d'entrée/sortie (14) avec un écran pour l'affichage des données de patient et/ou des paramètres de fonctionnement attribués aux données de patient du dispositif médical (10), dans lequel
un utilisateur peut, au moyen d'une entrée en utilisant le dispositif d'entrée/sortie (14), faire en sorte que les données de patient stockées dans le dispositif de stockage (13) et/ou les paramètres de fonctionnement attribués aux données de patient du dispositif médical (10) qui sont stockés dans le dispositif de stockage (13) soient affichés sur l'écran du dispositif d'entrée/sortie (14) uniquement si le dispositif médical (10) a déterminé que l'authentification est réussie.

9. Système de traitement (100) selon la revendication 2, dans lequel le dispositif médical (10) comprend un dispositif de stockage (13) dans lequel est stocké un numéro d'identification individuel qui identifie clairement le dispositif médical (10),
le dispositif médical (10) est configuré, dans le signal qui contient un signal qui correspond au signal acoustique, pour envoyer en outre un signal qui correspond au numéro d'identification individuel stocké dans le dispositif de stockage (13), et
le dispositif d'authentification portable (20) comprend un dispositif d'entrée/sortie (24) avec un écran et est configuré pour transmettre le numéro d'identification individuel sur l'écran après réception du signal envoyé par le dispositif médical (10).

10. Système de traitement (100) selon la revendication 9, dans lequel
des données de patient et/ou des paramètres de fonctionnement attribués aux données de patient du dispositif médical (10) qui avaient été enregistrés pendant un traitement du patient peuvent être stockés dans le dispositif de stockage (13), et
le dispositif médical (10) comprend en outre un dispositif d'entrée/sortie (14) avec un écran pour l'affichage des données de patient et/ou des paramètres de fonctionnement attribués aux données de patient du dispositif médical (10), dans lequel
un utilisateur peut, au moyen d'une entrée en utilisant le dispositif d'entrée/sortie (14), faire en sorte que les données de patient stockées dans le dispositif de stockage (13) et/ou les paramètres de fonctionnement attribués aux données de patient du dispositif médical (10) qui sont stockés dans le dispositif de stockage (13) soient affichés sur l'écran du dispositif d'entrée/sortie (14) uniquement si le dispositif médical (10) a déterminé que l'authentification est réussie.

11. Système de traitement (100) selon l'une des revendications précédentes, dans lequel le dispositif d'authentification portable (20) est conçu en tant que téléphone mobile ou en tant qu'ordinateur portable et/ou le dispositif médical (10) est un dispositif de dialyse qui est configuré pour la réalisation d'un traitement de dialyse.

12. Procédé destiné à vérifier l'authentification d'un dispositif d'authentification portable (20) pour un système de traitement (100) selon la revendication 1, comprenant :
la transmission d'un signal acoustique par le dispositif médical (10),
la réception du signal acoustique par le dispositif d'authentification portable (20), la production, par le dispositif d'authentification portable (20), en se basant sur le signal acoustique reçu, d'un signal qui contient un signal qui correspond au signal acoustique reçu, et l'envoi sans fil du signal produit au dispositif médical (10) par le dispositif d'authentification portable (20), et
le fait de déterminer, par le dispositif médical (10), selon qu'il reçoit ou non le signal qui contient le signal qui correspond au signal acoustique reçu depuis le dispositif d'authentification portable (20), si le dispositif d'authentification portable (20) se trouve ou non dans une position où une communication acoustique entre le dispositif médical (10) et le dispositif d'authentification portable (20) est possible,
l'envoi sans fil, par le dispositif médical (10) au dispositif d'authentification portable (20), d'un signal d'interrogation pour l'interrogation d'informations d'authentification,
l'envoi cyclique et/ou l'envoi après réception du signal d'interrogation, par le dispositif d'authentification portable (20), d'un signal d'authentification qui contient des informations qui identifient le dispositif d'authentification portable (20),
la réception sans fil, par le dispositif médical (10), du signal d'authentification depuis le dispositif d'authentification portable (20), et
la réalisation, par le dispositif médical (10), d'une authentification par laquelle est vérifié si le signal d'authentification est attribué ou non à un utilisateur enregistré dans une base de données d'autorisation ou à un dispositif d'authentification portable (20) enregistré dans la base de données d'autorisation, et si le dispositif d'authentification portable (20) se trouve ou non dans la position où la communication acoustique entre le dispositif médical (10) et le dispositif d'authentification portable (20) est possible, et le fait de déterminer, par le dispositif médical (10), que l'authentification est réussie lorsque le signal d'authentification est attribué à un utilisateur enregistré dans la base de données d'autorisation ou à un dispositif d'authentification portable (20) enregistré dans la base de données d'autorisation, et que le dispositif d'authentification portable (20) se trouve dans la position où la communication acoustique entre le dispositif médical (10) et le dispositif d'authentification portable (20) est possible.

13. Procédé destiné à vérifier l'authentification d'un dispositif d'authentification portable (20) pour un système de traitement (100) selon la revendication 2, comprenant :
l'envoi sans fil, par le dispositif médical (10), d'un signal qui contient un signal qui correspond à un signal acoustique,
la réception, par le dispositif d'authentification portable (20), du signal qui correspond au signal acoustique, la production, par le dispositif d'authentification portable (20), en se basant sur le signal acoustique reçu, d'un signal acoustique qui correspond au signal qui correspond au signal acoustique, et la transmission du signal acoustique produit au dispositif médical (10), et
le fait de déterminer, par le dispositif médical (10), selon qu'il reçoit ou non le signal acoustique, si le dispositif d'authentification portable (20) se trouve ou non dans une position où une communication acoustique entre le dispositif médical (10) et le dispositif d'authentification portable (20) est possible,
l'envoi sans fil, par le dispositif médical (10) au dispositif d'authentification portable (20), d'un signal d'interrogation pour l'interrogation d'informations d'authentification,
l'envoi cyclique et/ou l'envoi après réception du signal d'interrogation, par le dispositif d'authentification portable (20), d'un signal d'authentification qui contient des informations qui identifient le dispositif d'authentification portable (20),
la réception sans fil, par le dispositif médical (10), du signal d'authentification depuis le dispositif d'authentification portable (20), et
la réalisation, par le dispositif médical (10), d'une authentification par laquelle est vérifié si le signal d'authentification est attribué ou non à un utilisateur enregistré dans une base de données d'autorisation ou à un dispositif d'authentification portable (20) enregistré dans la base de données d'autorisation, et si le dispositif d'authentification portable (20) se trouve ou non dans la position où la communication acoustique entre le dispositif médical (10) et le dispositif d'authentification portable (20) est possible, et le fait de déterminer, par le dispositif médical (10), que l'authentification est réussie lorsque le signal d'authentification est attribué à un utilisateur enregistré dans la base de données d'autorisation ou à un dispositif d'authentification portable (20) enregistré dans la base de données d'autorisation, et que le dispositif d'authentification portable (20) se trouve dans la position où la communication acoustique entre le dispositif médical (10) et le dispositif d'authentification portable (20) est possible.

14. Procédé destiné à vérifier l'authentification d'un dispositif d'authentification portable (20) pour un système de traitement (100) selon la revendication 3, comprenant :
l'envoi sans fil, par le dispositif d'authentification portable (20), d'un signal qui contient un signal qui correspond à un signal acoustique,
la réception, par le dispositif médical (10), du signal qui contient le signal qui correspond au signal acoustique, la production, par le dispositif médical (10), en se basant sur le signal reçu, d'un signal acoustique qui correspond au signal qui correspond au signal acoustique, et la transmission, par le dispositif médical (10) au dispositif d'authentification portable (20), du signal acoustique produit,
l'envoi sans fil, par le dispositif d'authentification portable (20) au dispositif médical (10), d'un signal qui indique que le dispositif d'authentification portable (20) a reçu le signal acoustique lorsque le dispositif d'authentification portable (20) a reçu le signal acoustique depuis le dispositif médical (10), et
le fait de déterminer, par le dispositif médical (10), selon qu'il reçoit ou non le signal qui indique que le dispositif d'authentification portable (20) a reçu le signal acoustique, si le dispositif d'authentification portable (20) se trouve ou non dans une position où une communication acoustique entre le dispositif médical (10) et le dispositif d'authentification portable (20) est possible,
l'envoi sans fil, par le dispositif médical (10) au dispositif d'authentification portable (20), d'un signal d'interrogation pour l'interrogation d'informations d'authentification,
l'envoi cyclique et/ou l'envoi après réception du signal d'interrogation, par le dispositif d'authentification portable (20), d'un signal d'authentification qui contient des informations qui identifient le dispositif d'authentification portable (20),
la réception sans fil, par le dispositif médical (10), du signal d'authentification depuis le dispositif d'authentification portable (20), et
la réalisation, par le dispositif médical (10), d'une authentification par laquelle est vérifié si le signal d'authentification est attribué ou non à un utilisateur enregistré dans une base de données d'autorisation ou à un dispositif d'authentification portable (20) enregistré dans la base de données d'autorisation, et si le dispositif d'authentification portable (20) se trouve ou non dans la position où la communication acoustique entre le dispositif médical (10) et le dispositif d'authentification portable (20) est possible, et le fait de déterminer, par le dispositif médical (10), que l'authentification est réussie lorsque le signal d'authentification est attribué à un utilisateur enregistré dans la base de données d'autorisation ou à un dispositif d'authentification portable (20) enregistré dans la base de données d'autorisation, et que le dispositif d'authentification portable (20) se trouve dans la position où la communication acoustique entre le dispositif médical (10) et le dispositif d'authentification portable (20) est possible.

15. Procédé destiné à vérifier l'authentification d'un dispositif d'authentification portable (20) pour un système de traitement (100) selon la revendication 4, comprenant :
la transmission d'un signal acoustique prédéfini, de préférence d'une suite de notes prédéfinie, par le dispositif d'authentification portable (20), et
le fait de déterminer, par le dispositif médical (10), selon qu'il reçoit ou non le signal acoustique prédéfini, si le dispositif d'authentification portable (20) se trouve ou non dans une position où une communication acoustique entre le dispositif médical (10) et le dispositif d'authentification portable (20) est possible,
l'envoi sans fil, par le dispositif médical (10) au dispositif d'authentification portable (20), d'un signal d'interrogation pour l'interrogation d'informations d'authentification,
l'envoi cyclique et/ou l'envoi après réception du signal d'interrogation, par le dispositif d'authentification portable (20), d'un signal d'authentification qui contient des informations qui identifient le dispositif d'authentification portable (20),
la réception sans fil, par le dispositif médical (10), du signal d'authentification depuis le dispositif d'authentification portable (20), et
la réalisation, par le dispositif médical (10), d'une authentification par laquelle est vérifié si le signal d'authentification est attribué ou non à un utilisateur enregistré dans une base de données d'autorisation ou à un dispositif d'authentification portable (20) enregistré dans la base de données d'autorisation, et si le dispositif d'authentification portable (20) se trouve ou non dans la position où la communication acoustique entre le dispositif médical (10) et le dispositif d'authentification portable (20) est possible, et le fait de déterminer, par le dispositif médical (10), que l'authentification est réussie lorsque le signal d'authentification est attribué à un utilisateur enregistré dans la base de données d'autorisation ou à un dispositif d'authentification portable (20) enregistré dans la base de données d'autorisation, et que le dispositif d'authentification portable (20) se trouve dans la position où la communication acoustique entre le dispositif médical (10) et le dispositif d'authentification portable (20) est possible.
